# EUROPEAN PATENT SPECIFICATION

(11) **EP 1 560 915 B1**
(45) Date of publication and mention of the grant of the patent: **04.03.2009**
(21) Application number: 03774653.4
(22) Date of filing: 09.10.2003
(51) Int. Cl.: C12N 5/00, G01N 33/68

(54) **ASSAY METHODS FOR STATE-DEPENDENT CALCIUM CHANNEL AGONISTS/ANTAGONISTS**
TESTVERFAHREN FÜR ZUSTANDSABHÄNGIGE CALCIUMKANAL-AGONISTEN/-ANTAGONISTEN
METHODES DE DOSAGE D'AGONISTES/ANTAGONISTES DE CANAUX CALCIQUES SELON L'ETAT

(30) Priority: 10.10.2002 US 418017 P
(43) Date of publication of application: 10.08.2005
(73) Proprietor: Merck & Co., Inc., Rahway, New Jersey 07065-0907 (US)
(72) Inventor: XIA, Menghang, Rahway, NJ 07065-0907 (US); CONNOLLY, Thomas, M., Rahway, NJ 07065-0907 (US); BENNETT, Paul, B., Jr., Rahway, NJ 07065-0907 (US); COHEN, Charles, J., Rahway, NJ 07065-0907 (US)
(74) Representative: Horgan, James Michael Frederic
(86) International application number: PCT/US2003/031822
(87) International publication number: WO 2004/033647

(56) References cited:
- US-A- 5 043 457
- US-A- 5 962 477
- JAVORS MARTIN A ET AL: "Partial characterization of K+-induced increase in (Ca-2+)-cyt and GnRH release in GT1-7 neurons" BRAIN RESEARCH, vol. 694, no. 1-2, 1995, pages 49-54, XP002401336 ISSN: 0006-8993
- SHIAKA YOSHITSUGU ET AL: "Basic fibroblast growth factor increases functional L-type Ca-2+ channels in fetal rat hippocampal neurons: Implications for neurite morphogenesis in vitro" JOURNAL OF NEUROSCIENCE, vol. 16, no. 20, 1996, pages 6476-6489, XP002401337 ISSN: 0270-6474
- MARCHETTI CARLA ET AL: "High affinity block by nimodipine of the internal calcium elevation in chronically depolarized rat cerebellar granule neurons" NEUROSCIENCE LETTERS, vol. 207, no. 2, 1996, pages 77-80, XP002401338 ISSN: 0304-3940
- VOITENKO N V ET AL: "Effect of streptozotocin-induced diabetes on the activity of calcium channels in rat dorsal horn neurons" NEUROSCIENCE, vol. 95, no. 2, 1 December 2000 (2000-12-01), pages 519-524, XP002401339 ISSN: 0306-4522
- YASUTSUNE TORU ET AL: "Vasorelaxation and inhibition of the voltage-operated Ca2+ channels by FK506 in the porcine coronary artery" BRITISH JOURNAL OF PHARMACOLOGY, vol. 126, no. 3, February 1999 (1999-02), pages 717-729, XP002401340 ISSN: 0007-1188
- BARGER S W: "Complex influence of the L-type calcium-channel agonist BAYK8644(racemic) on N-methyl-D-aspartate responses and neuronal survival" NEUROSCIENCE, vol. 89, no. 1, March 1999 (1999-03), pages 101-108, XP002401341 ISSN: 0306-4522
- NUMANN R ET AL: "High-throughput screening strategies for cardiac ion channels" TRENDS IN CARDIOVASCULAR MEDICINE, ELSEVIER SCIENCE, NEW YORK, NY, US, vol. 11, no. 2, 2001, pages 54-59, XP002324417 ISSN: 1050-1738

## Description

### FIELD OF THE INVENTION

The present invention is directed to methods and cells for studying the effect of candidate compounds on the activity of calcium channels. The methods utilize cells that express a calcium channel of interest and which express a potassium channel. The engineered cells allow for fine control of the membrane potential of the cells, which, in turn, provide a high resolution assay for studying the effects of targeted compounds at various states of the calcium channel.

### BACKGROUND OF THE INVENTION

Certain molecular events in eukaryotic cells depend on the existence or magnitude of an electric potential gradient across the plasma (i.e., outer) membrane of the cells. Among the more important of such events is the movement of ions across the plasma membrane through voltage-gated ion channels. Voltage-gated ion channels form transmembrane pores that open in response to changes in cell membrane potential and allow ions to pass through the membrane. Voltage-gated ion channels have many physiological roles. They have been shown to be involved in maintaining cell membrane potentials and controlling the repolarization of action potentials in many types of cells (Bennett et al., 1993, Cardiovascular Drugs & Therapy 7:195-202; Johnson et al., 1999, J. Gen. Physiol. 113:565-580; Bennett & Shin, "Biophysics of voltage-gated sodium channels," in Cardiac Electrophysiology: From Cell to Bedside, 3rd edition, D. Zipes & J. Jalife, eds., 2000, W.B. Saunders Co., pp.67-86; Bennett & Johnson, "Molecular physiology of cardiac ion channels," Chapter 2 in Basic Cardiac Electrophysiology and Pharmacology, 1st edition, A. Zasa & M. Rosen, eds., 2000, Harwood Academic Press, pp. 29-57). Moreover, mutations in sodium, calcium, or potassium voltage-gated ion channel genes leading to defective channel proteins have been implicated in a variety of disorders including the congenital long QT syndromes, ataxia, migraine, muscle paralysis, deafness, seizures, and cardiac conduction diseases, to name a few (Bennett et al., 1995, Nature 376:683-685; Roden et al., 1995, J. Cardiovasc. Electrophysiol. 6:1023-1031; Kors et al., 1999, Curr. Opin. Neurol. 12:249-254; Lehmann et al., 1999, Physiol. Rev. 79:1317-1372; Holbauer & Heufelder, 1997, Eur. J. Endocrinol. 136:588-589; Naccarelli & Antzelevitch, 2000, Am. J. Med. 110:573-581).

Several types of voltage-gated ion channels exist. Voltage-gated potassium channels establish the resting membrane potential and modulate the frequency and duration of action potentials in neurons, muscle cells, and secretory cells. Following depolarization of the membrane potential, voltage-gated potassium channels open, allowing potassium efflux and thus membrane repolarization. This behavior has made voltage-gated potassium channels important targets for drug discovery in connection with a variety of diseases. Dysfunctional voltage-gated potassium channels have been implicated in a number of diseases and disorders. Wang et al., 1998, Science 282:1890-1893 have shown that the voltage-gated potassium channels KCNQ2 and KCNQ3 form a heteromeric potassium ion channel known as the "M-channel." Mutations in KCNQ2 and KCNQ3 in the M-channel are responsible for causing epilepsy (Biervert et al., 1998, Science 279:403-406; Singh et al., 1998, Nature Genet. 18:25-29; Schroeder et al., Nature 1998, 396:687-690).

Voltage-gated sodium channels are transmembrane proteins that are essential for the generation of action potentials in excitable cells (Catterall, 1993, Trends Neurosci. 16:500-506). In mammals, voltage-gated sodium channels consist of a macromolecular assembly of α and β subunits with the α subunit being the pore-forming component. α subunits are encoded by a large family of related genes, with some α subunits being present in the central nervous system (Noda et al., 1986, Nature 322:826-828; Auld et al., 1988, Neuron 1:449-461; Kayano et al., 1988, FEBS Lett. 228:187-194) and others in muscle (Rogart et al., 1989, Proc. Natl. Acad. Sci. USA 86:8170-8174; Trimmer et al., 1989, Neuron 3:33-49).

Voltage-gated calcium channels are transmembrane proteins that in the open configuration allow the passive flux of Ca2+ ions across the plasma membrane, down the electrochemical gradient. They mediate various cell functions, including excitation-contraction coupling, signal transduction, and neurotransmitter release. Three major classes of calcium channel antagonists including the dihydropyridines, benzothiazepines and phenylalkylamines have been widely used clinically in the treatment of cardiovascular diseases. These drugs antagonize the L-type calcium channels found throughout the body, including the cardiovascular system. Calcium channels are allosteric proteins that undergo changes in conformational state. The distinct conformational states of these proteins have different affinities for ligands, including these antagonists. Membrane potential is an allosteric effector of these conformational changes in ion channel proteins. The potency of inhibition by these calcium channel antagonists is dependent on the state of the calcium channel. Previously studies on state-dependent interactions of these antagonists were identified through voltage clamp (1), radioligand binding (2) and cell based, e.g. smooth muscle contraction (3) studies. While each of these methods yields valuable information each has its drawbacks in terms of information content or throughput, respectively.

Calcium channels are membrane-spanning, multi-subunit proteins that allow controlled entry of Ca+2 ions into cells from the extracellular fluid. Cells throughout the animal kingdom, and at least some bacterial, fungal and plant cells, possess one or more types of calcium channel.

The most common type of calcium channel is voltage dependent. Most "excitable" cells in animals, such as neurons of the central nervous system (CNS), peripheral nerve cells and muscle cells, including those of skeletal muscles, cardiac muscles, and venous and arterial smooth muscles, have voltage-dependent calcium channels. "Opening" of a voltage-dependent channel to allow an influx of Ca+2 ions into the cells requires a depolarization to a certain level of the potential difference between the inside of the cell bearing the channel and the extracellular environment bathing the cell. The rate of influx of Ca+2 into the cell depends on this potential difference.

Multiple types of calcium channels have been identified in mammalian cells from various tissues, including skeletal muscle, cardiac muscle, lung, smooth muscle and brain, [see, e.g., Bean, B. P. (1989) Ann. Rev. Physiol. 51:367-384 and Hess, P. (1990) Ann. Rev. Neurosci. 56:337]. The different types of calcium channels have been broadly categorized into five classes, L-, T-, N-, P/Q and R-type, distinguished by current kinetics, holding potential sensitivity and sensitivity to calcium channel agonists and antagonists.

Current methods of drug discovery often involve assessing the biological activity (i.e., screening) of tens or hundreds of thousands of compounds in order to identify a small number of those compounds having a desired activity. In many high throughput screening programs, it is desirable to test as many as 50,000 to 100,000 compounds per day. Unfortunately, current methods of assaying the activity of voltage-gated ion channels are ill suited to the needs of a high throughput screening program. Current methods often rely on electrophysiological techniques. Standard electrophysiological techniques involve "patching" or sealing against the cell membrane with a glass pipette followed by suction on the glass pipette, leading to rupture of the membrane patch (Hamill et al., 1981, Pflugers Arch. 391:85-100). This has limitations and disadvantages. Accessing the cell interior may alter the cell's response properties. The high precision optical apparatuses necessary for micromanipulating the cells and the pipettes make simultaneous recording from more than a few cells at a time impossible. Given these difficulties, the throughput that can be achieved with electrophysiological techniques falls far short of that necessary for high throughput screening.

Various techniques have been developed as alternatives to standard methods of electrophysiology. For example, radioactive flux assays have been used in which cells are exposed with a radioactive tracer (e.g., 86Rb⁺, ²²Na⁺, [¹⁴C]-guanidinium and 45Ca) and the flux of the radio-labled ion is monitored. Cells loaded with the tracer are exposed to compounds and those compounds that either enhance or diminish the efflux of the tracer are identified as possible activators or inhibitors of ion channels in the cells' membranes.

Assays that measure the change in a cell's membrane potential due to the change in activity of an ion channel have been developed. Such assays often employ voltage sensitive dyes that redistribute between the extracellular environment and the cell's interior based upon a change in membrane potential and that have a different fluorescence spectrum depending on whether they are inside or outside the cell. A related assay method uses a pair of fluorescent dyes capable of fluorescence resonance energy transfer to sense changes in membrane potential. For a description of this technique, see González & Tsien, 1997, Chemistry & Biology 4:269-277. See also González & Tsien, 1995, Biophys. J. 69:1272-1280 and U.S. Patent No. 5,661,035. Other methods employ ion selective indicators such as calcium dependent fluorescent dyes to monitor changes in Ca²⁺ influx during opening and closing of calcium channels.

Ideally, methods of screening against voltage-gated ion channels require that the transmembrane potential of the cells being assayed be controlled and/or that the ion channels studied be cycled between open and closed states. This has been done in various ways. In standard electrophysiological techniques, the experimental set-up allows for direct manipulation of membrane potential by the voltage clamp method (Hodgkin & Huxley, 1952, J. Physiol. (Lond.) 153:449-544), e.g., changing the applied voltage. In other methods, changing the extracellular K⁺ concentration from a low value (e.g., 5 mM) to a higher value (e.g., 70-80 mM) results in a change in the electrochemical potential for K⁺ due to the change in the relative proportion of intracellular and extracellular potassium. This results in a change in the transmembrane electrical potential towards a more depolarized state. This depolarization can activate many voltage-gated ion channels, e.g., voltage-gated calcium, sodium, or potassium channels. This technique has been used to determine voltage-gated calcium channel function under certain conditions (Javors et al., 1995, Brain Res 694:49-54; Shiaka et al., 1996, J. Neurosci 16:6476-6489; Marchetti et al., 1996, Neurosci Lett 207:77-80; Voitenko et al., 2000, Neurosci 95:519-524; Yasutsune et al., 1999, J. Pharmacol 126:717-729). Alternatively, Na+ channels can be induced into an open conformation by the use of toxins such as veratridine or scorpion venom (Strichartz et al., 1987, Ann. Rev. Neurosci. 10:237-267; Narahashi & Harman, 1992, Meth. Enzymol. 207:620-643). While sometimes effective, such experimental manipulations may alter the channel pharmacology, can be awkward to perform, and can lead to artifactual disturbances in the system being studied.

Electrical field stimulation (EFS) has been used to activate ion channels. In this approach, membrane potential is altered but not controlled. The uncertainty and lack of control of membrane potential make EFS a less than optimal method for the study of ion channels.

HEK293 cells have been grown on a silicon chip made up of an array of field-effect transistors. Some of the cells were positioned over the gate region of the transistors, thus having portions of their plasma membranes overlying the source and the drain. When a patch pipette in such cells manipulated the intracellular voltage, Maxi-K potassium channels in the cells' plasma membranes were opened. This led to current flow in the region between the cells' membrane and the transistor. This current flow modulated the source-drain current, which could be detected by an appropriate device. The chip plus cells was said to have potential as a sensor and as a prototype for neuroprosthetic devices. See Straub et al., 2001, Nature Biotechnol. 19:121-124; Neher, 2001, Nature Biotechnol. 19:114.

### SUMMARY OF THE INVENTION

The present invention is directed to methods of identifying activators and inhibitors of voltage-gated ion channels, and specifically calcium ion channels. The methods employ cells transformed to express a voltage-gated calcium ion channel of interest and an inward rectifier potassium channel. The addition of the potassium channel allows for the fine control of the membrane potential of the cells. Manipulation of the extracellular potassium concentration controls the membrane potential which in turn affects the open/close state transitions of the voltage-gated ion channels. This allows for more convenient, more precise manipulation of these transitions, and, coupled with efficient methods of detecting ion flux, results in methods that are especially suitable for high throughput screening in order to identify substances that are channel state dependent modulators of voltage-gated ion channels.

According to a specific embodiment, the present invention describes the state-dependent interactions of the calcium channel antagonists directly in a functional cell-based FLIPR (Fluorometric Imaging Plate Reader) assay, which measures calcium influx through a voltage-dependent calcium channel (VDCC). The cell line used in this embodiment has a stably transfected L-type calcium channel, the α1 C subunit. It also was transfected with the Kir 2.3 inward rectifier K channel, which allows for control of cell membrane potential through alteration of extracellular [K⁺]o. Preincubation of the cells for 10 min in 30 mM [K⁺]o partially depolarizes the cells. The inhibitory effect of calcium channel antagonists on calcium influx in response to a high [K⁺]o depolarization (final [K⁺]o 85.8 mM) was shifted to the left compared with that observed for cells in normal, physiological [K⁺]o (5.8 mM). The ratio of IC₅₀ values between the potencies for the antagonists tested in the normally polarized and depolarized cells was 4 to 20-fold. The results suggest that the interaction of these calcium channel antagonists with the channel expressing cells is dependent upon the state of the channel, which is modulated by changes in membrane potential. The state dependent assay demonstrated in these studies is useful for evaluating state dependent inhibitory potency of a large number of samples and can be used to identify state-dependent calcium channel antagonists.

### BRIEF DESCRIPTION OF THE DRAWINGS

**Figure 1** shows immunostaining of alpha 1C subunit in the wild type HEK 293 cells and cells stably transfected with the L-type α1C channel (C1-6-37-3).
**Figure 2** shows immunostaining of Kir2.3 subunit in the wild type HEK293 cells and the cells stably transfected with the L-type α1C channel (C1-6-37-3).
**Figure 3** is a graph showing the relationship between extracellular potassium ([K]o) and cell membrane potential. Three situations are shown. One is the prediction of the Nernst equation for a perfectly K-selective membrane. The other curves show the effects of partial permeability by other ions, Na⁺ and/or Cl⁻. Membrane potential can be set in a non-voltage clamped cell by adjusting external potassium.
A cell line expressing an inward rectifier K channel (Kir2.3) to set the resting membrane potential will permit control of membrane resting potential by extracellular potassium.
**Figure 4** is a graph demonstrating the dose-response relationship for K⁺-stimulated calcium influx in wild type HEK 293 cells and cells stably transfected with the L-type α1C channel (C1-6-37-3).
**Figure 5** is a graph demonstrating a comparison of nimodipine and mibefradil inhibition curves in K⁺-stimulated calcium influx in C1-6-37-3 cells under resting condition (5.8 mM K =-65 mV).
**Figure 6** is a graph representing the nimodipine inhibition curve stimulated by K⁺ (final 85.8 mM) either in 30 mM K⁺ (depolarized condition, -28 mV) or 5.8 mM K⁺ (resting condition, -65 mV).
**Figure 7** is summary table of IC50 (nM) values for calcium channel antagonists in 30 mM K⁺ (depolarized condition, -28 mV) and 5.8 mM K⁺ (resting condition, -65 mV).

### DETAILED DESCRIPTION OF THE INVENTION

Without intending to bound by any theory, voltage gated calcium channels open as a function of membrane potential such that the probability of opening increases with membrane depolarization. Voltage gated calcium channels inactivate (close / desensitize) as a function of membrane potential such that the probability of inactivation increases with membrane depolarization. These steady state voltage dependent processes overlap. Changes in membrane potential populate different conformational states of these channels (closed, open or inactivated). Drug binding to voltage gated calcium channels is often channel state dependent such that more or less binding occurs depending upon the state occupied. Control of membrane potential, permits channels to be manipulated into various states. This membrane potential control is typically achieved by voltage clamp electrophysiology methods, but this method is not at present amenable to high throughput drug screening.

Specifically exemplified herein is an assay to determine state-dependent drug-calcium channel interactions using a cell line that co-expresses a potassium channel (Kir2.3) that determines the resting membrane potential of the cells as a function of the external potassium ion concentration ([K]o) and a voltage gated calcium channel. Co-expressed in these cells is the L-type voltage gated calcium channel complex (alpha1C, alpha2-delta, beta2a). Potassium is used in a two step manner in this assay. First it is used to set the resting membrane potential (Vm) during antagonist incubation. Two conditions were selected for illustration purposes, polarized and depolarized resting conditions. In the polarized resting condition, cells are incubated in 5.8 mM [K]o to set the membrane potential to -65 mV (Vm as a function of [K]o). Drugs exposed to these cells will bind to calcium channels primarily in the closed, rested, low affinity state. In order to reveal higher affinity states of the calcium channels, the cells are incubated in 30 mM [K]o, in order to chronically and partially depolarize them to -28 mV during drug exposure. This change in the membrane potential, shifts the calcium channels into the higher affinity inactivated states and antagonist binding is enhanced. Upon establishing these two different conditions for drug exposure, channels are then forced to open by further depolarization to near 0 mV by exposure to 85.8 mM [K]o. Opening of these channels normally under control, non-antagonist exposed conditions, allows calcium influx into the cells. This calcium influx is detected using a calcium sensitive dye (eg Fluo-3, Fluo-4, Fura2, etc.). If the calcium influx is diminished by exposure to antagonists, this will be detected when compared to the control condition. In some cases, antagonists will bind with greater affinity to the channels in the depolarized (30 mM [K]o) condition. In these cases, the same drug will appear more potent under these depolarized assay conditions. This approach creates a novel high throughput calcium channels assay system that is capable of detecting and measuring calcium channel state dependent drug interactions as have been described using low throughput voltage clamp measures on single cells.

This foregoing approach and the referenced cells have been tested using conventional voltage- and current- clamp methods, and the membrane potential changes as a function [K]o and the state dependent calcium current and drug affinities have been confirmed experimentally. The foregoing approach can be modified as taught herein to study state-dependencies of agonists/antagonists for many different types of ion channels.

Also disclosed is a substrate upon which living eukaryotic cells, preferably mammalian cells, are present where the cells express voltage-gated calcium ion channels in their plasma membranes. Upon application of varying concentrations of extracellular calcium, voltage-gated ion channels either open or close, thereby modulating the flow of at least one type of ion through the plasma membranes of the cells. This modulation of ion flow, or a change in membrane potential that results from the modulation of ion flow, is detected, either directly or indirectly, preferably by the use of fluorescent indicator compounds in the cells. Collections of substances, e.g., combinatorial libraries of small organic molecules, natural products, phage display peptide libraries, etc., are brought into contact with the voltage-gated ion channels in the plasma membranes of the cells and those substances that are able to affect the modulation of ion flow are identified. In this way, the present invention provides methods of screening for activators and inhibitors of voltage-gated ion channels, particularly calcium channels. Such activators and inhibitors are expected to be useful as pharmaceuticals or as lead compounds from which pharmaceuticals can be developed by the usual processes of drug development, e.g., medicinal chemistry.

Accordingly, the present invention provides a method for identifying modulators of the activity of a voltage-gated calcium ion channel comprising:
(a) providing cells expressing the voltage-gated calcium ion channel and expressing an inward rectifying potassium channel;
(b) dividing the cells into group 1 and group 2;
(c) changing extracellular potassium concentration of the group 2;
(c) exposing the cells of groups 1 and 2 to a substance of interest;
(d) depolarizing the cells of groups 1 and 2 while monitoring ion flux through the voltage-gated calcium ion channel;
(c) comparing the ion flow through the voltage-gated calcium ion channel in groups 1 and 2;
   where a difference in the ion flow through the voltage-gated calcium ion channel in groups 1 and 2 indicates that the substance is a modulator of the voltage-gated channels, and
   where the potency of the modulator is affected by the state of the voltage-gated calcium ion channel.

For the sake of simplicity, the above methods are described in terms of "a" voltage-gated ion channel although those skilled in the art will understand that in actual practice the cells will express a plurality of the voltage-gated ion channels for which modulators are sought. Generally, each cell will express at least 10², 103, 10⁴, 10⁵, 10⁶ or more molecules of the voltage-gated ion channel. Also, ion flow will be monitored through the plurality of the voltage-gated ion channels rather than through a single voltage-gated ion channel. Similarly, the methods will generally be practiced by employing a plurality of cells, even though the methods are described above in terms of "a" cell.

Generally, the methods of the present invention will be carried out on a substrate that is a modified version of a standard multiwell tissue culture plate or microtiter plate.

The skilled person will recognize that it is generally beneficial to run controls together with the methods described herein. For example, it will usually be helpful to have a control in which the substances are tested in the methods against cells that preferably are essentially identical to the cells that are used in the methods except that these cells would not express the voltage-gated ion channels of interest. In this way it can be determined that substances which are identified by the methods are really exerting their effects through the voltage-gated ion channels of interest rather than through some unexpected non-specific mechanism. One possibility for such control cells would be to use non-recombinant parent cells where the cells of the actual experiment express the voltage-gated ion channels of interest due to the recombinant expression of those voltage-gated ion channels of interest.

Other types of controls would involve taking substances that are identified by the methods of the present invention as activators or inhibitors of voltage-gated ion channels of interest and testing those substances in the methods of the prior art in order to confirm that those substances are also activators and inhibitors when tested in those prior art methods.

One skilled in the art would recognize that, where the present invention involves comparing control values for the flow of ions to test values for the flow of ions and determining whether the control values are greater or less than the test values, a non-trivial difference is sought. For example, if in the methods of identifying inhibitors, the control value were found to be 1% greater than the test value, this would not indicate that the substance is an inhibitor. Rather, one skilled in the art would attribute such a small difference to normal experimental variance. What is looked for is a significant difference between control and test values. For the purposes of this invention, a significant difference fulfills the usual requirements for a statistically valid measurement of a biological signal. For example, depending upon the details of the experimental arrangement, a significant difference might be a difference of at least 10%, preferably at least 20%, more preferably at least 50%, and most preferably at least 100%.

One skilled in the art would understand that the cells that give rise to the control values need not be physically the same cells that give rise to the test values, although that is possible. What is necessary is that the cells that give rise to the control values be substantially the same type of cell as the cells that give rise to the test values. A cell line that has been transfected with and expresses a certain voltage-gated ion channel could be used for both the control and test cells. Large numbers of such cells could be grown and a portion of those cells could be exposed to the substance and thus serve as the cells giving rise to the test value for ion flow while a portion would not be exposed to the substance and would thus serve as the cells giving rise to the control value for ion flow. No individual cell itself would be both control and test cell but the virtual identity of all the cells in the cell line ensures that the methods would nevertheless be reliable.

"Substances" can be any substances that are generally screened in the pharmaceutical industry during the drug development process. For example, substances may be low molecular weight organic compounds (*e*.*g*., having a molecular weight of less than about 1,000 daltons); RNA, DNA, antibodies, peptides, or proteins.

The conditions under which cells are exposed to substances in the methods described herein are conditions that are typically used in the art for the study of protein-ligand interactions: *e*.*g*., physiological pH; salt conditions such as those represented by such commonly used buffers as PBS or in tissue culture media; a temperature preferably of about 18°C to about 45°C; incubation times of from several seconds to several hours. Generally, the cells are present in wells in the substrate and the substances are added directly to the wells, optionally after first washing away the media in the wells.

Determining the values of ion flux in the methods of the present invention can be accomplished through the use of fluorescent indicator compounds. One type of fluorescent indicator compound is sensitive to the level of intracellular calcium ions in the cells used in the present invention. This type of fluorescent indicator compound can be used when the methods are directed to those voltage-gated ion channels whose activity results in a change in intracellular calcium levels. Such voltage-gated ion channels include not only voltage-gated calcium channels but also other types of voltage-gated ion channels where the activity of those channels is naturally or can be coupled to changes in intracellular calcium levels. Many types of voltage-gated potassium channels can be so coupled. When using this approach to study a voltage-gated ion channel of interest that is not a voltage-gated calcium channel, it may be desirable to engineer the cells employed so as to recombinantly express voltage-gated calcium channels that are coupled to the voltage-gated ion channel of interest.

Fluorescent indicator compounds suitable for measuring intracellular calcium levels include various calcium indicator dyes (*e*.*g*., fura-2, fluo-3, fluo-4, indo-1, Calcium Green; see Veliçelebi et al., 1999, Meth. Enzymol. 294:20-47).

Calcium indicator dyes are substances which show a change in a fluorescent characteristic upon binding calcium, *e*.*g*., greatly increased intensity of fluorescence and/or a change in fluorescent spectra (*i*.*e*., a change in emission or excitation maxima). Fluo-3, fura-2, and indo-1 are commonly used calcium indicator dyes that were designed as structural analogs of the highly selective calcium chelators ethylene glycol-bis(β-aminoethyl ether) N,N,N',N'-tetraacetic acid (EGTA) and 1,2-bis(2-aminophenoxy) ethane-N,N,N',N'-tetraacetic acid (BAPTA). The fluorescence intensity from fluo-3 increases by more than 100-fold upon binding of calcium. While the unbound dye exhibits very little fluorescence, calcium-bound fluo-3 shows strong fluorescence emission at 526 nm. Fura-2 is an example of a dye that exhibits a change in its fluorescence spectrum upon calcium binding. In the unbound state, fura-2 has an excitation maximum of 362 nm. This excitation maximum shifts to 335 nm upon calcium binding, although there is no change in emission maximum. Binding of calcium to fura-2 can be monitored by excitation at the two excitation maxima and determining the ratio of the amount of fluorescence emission following excitation at 362 nm compared to the amount of fluorescence emission following excitation at 335 nm. A smaller ratio (*i*.*e*., less emission following excitation at 362 nm) indicates that more fura-2 is bound to calcium, and thus a higher internal calcium concentration in the cell.

The use of calcium indicator dyes entails loading cells with the dye, a process which can be accomplished by exposing cells to the membrane-permeable acetoxymethyl esters of the dyes. Once inside the plasma membrane of the cells, intracellular esterases cleave the esters, exposing negative charges in the free dyes. This prevents the free dyes from crossing the plasma membrane and thus leaves the free dyes trapped in the cells. Measurements of fluorescence from the dyes are then made, the cells are treated in such a way that the internal calcium concentration is changed (*e*.*g*., by exposing cells to an activator or inhibitor of a voltage-gated ion channel), and fluorescence measurements are again taken.

Fluorescence from the indicator dyes can be measured with a luminometer or a fluorescence imager. One preferred detection instrument is the Fluorometric Imaging Plate Reader (FLIPR) (Molecular Devices, Sunnyvale, CA). The FLIPR is well suited to high throughput screening using the methods of the present invention as it incorporates integrated liquid handling capable of simultaneously pipetting to 96 or 384 wells of a microtiter plate and rapid kinetic detection using a argon laser coupled to a charge-coupled device imaging camera.

A typical protocol for use of calcium indicator dyes would entail putting cells expressing a voltage-gated ion channel of interest into an appropriate substrate (*e*.*g*., clear, flat-bottom, black-wall 96 well plates) and allowing the cells to grow overnight in standard tissue culture conditions (*e*.*g*., 5% CO₂, 37°C). The cells are generally plated at a density of about 10,000 to 100,000 cells per well in appropriate growth medium. On the day of the assay, growth medium is removed and dye loading medium is added to the wells.

If the calcium indicator dye is fluo-3, *e*.*g*., dye loading medium could be prepared by solubilizing 50 µg of fluo-3-AM ester (Molecular Probes F-1242) in 22 µl DMSO to give a 2 mM dye stock. Immediately before loading the cells, 22 µl 20% pluronic acid (Molecular Probes P-3000) is added to the dye. The tube containing the dye is mixed with a vortex mixer. For one 96-well plate, 44 ml of the dye/pluronic acid solution is added to 10.5 ml of Hanks Balanced Salt Solution (Gibco/BRL Cat # 14025-076) with 20 mM HEPES (Gibco/BRL Cat # 1560-080), and 1% fetal bovine serum (Gibco/BRL Cat # 26140-087; not BSA)). The dye and the loading medium are mixed by repeated inversion (final dye concentration about 4 µM).

Growth medium can be removed from the cells by washing (wash medium is Hanks Balanced Salt Solution (Gibco/BRL Cat # 14025-076) with 20 mM HEPES (Gibco/BRL Cat # 1560-080), and 0.1% bovine serum albumin (Sigma Cat # A-9647; not FBS) two times, leaving 100 µl residual medium in the wells after the second wash. Then 100 µl of the dye in the loading medium is added to each well. The cells are then incubated for 60 minutes at 37°C to allow for dye loading.

Following dye loading, the cells in each well are washed for four times, then fluorescent measurements of the cells are taken prior to exposure of the cells to substances that are to be tested. The cells are then exposed to the substances and those substances that cause a change in a fluorescent characteristic of the dye are identified. The measuring instrument can be a fluorescent plate reader such as the FLIPR (Molecular Devices). Substances that cause a change in a fluorescent characteristic in the test cells but not the control cells are possible activators or inhibitors of the voltage-gated ion channel.

The exact details of the procedure outlined above are meant to be illustrative. One skilled in the art would be able to optimize experimental parameters (cell number, dye concentration, dye loading time, temperature of incubations, cell washing conditions, and instrument settings, etc.) by routine experimentation depending on the particular relevant experimental variables (*e*.*g*., type of cell used, identity of dye used). Several examples of experimental protocols that can be used are described in Veliçelebi et al., 1999, Meth. Enzymol. 294:20-47. Other suitable instrumentation and methods for measuring transmembrane potential changes via optical methods includes microscopes, multiwell plate readers and other instrumentation that is capable of rapid, sensitive ratiometric fluorescence detection. For example, the VIPR (Aurora Biosciences, San Diego, CA) is an integrated liquid handler and kinetic fluorescence reader for 96-well and greater multiwell plates. The VIPR reader integrates an eight channel liquid handler, a multiwell positioning stage and a fiber-optic illumination and detection system. The system is designed to measure fluorescence from a column of eight wells simultaneously before, during and after the introduction of liquid sample obtained from another microtiter plate or trough. The VIPR reader excites and detects emission signals from the bottom of a multiwell plate by employing eight trifurcated optical bundles (one bundle for each well). One leg of the trifurcated fiber is used as an excitation source, the other two legs of the trifurcated fiber being used to detect fluorescence emission. A ball lens on the end of the fiber increases the efficiency of light excitation and collection. The bifurcated emission fibers allow the reader to detect two emission signals simultaneously and are compatible with rapid signals generated by the FRET-based voltage dyes. Photomultiplier tubes then detect emission fluorescence, enabling sub-second emission ratio detection.

In particular embodiments, the calcium indicator dye is selected from the group consisting of: fluo-3, fura-2, fluo-4, fluo-5, calcium green-1, Oregon green, 488 BAPTA, SNARF-1, and indo-1.

In particular embodiments, the change in fluorescent characteristic is an increase in intensity of a fluorescence emission maximum. In other embodiments, the change in fluorescent characteristic is a shift in the wavelength of an absorption maximum.

In particular embodiments, the cells naturally express the voltage-gated ion channel of interest. In other embodiments, the cells do not naturally express the voltage-gated ion channel of interest but instead have been transfected with expression vectors that encode the voltage-gated ion channel of interest so that the cells recombinantly express the voltage-gated ion channel of interest. Transfection is meant to include any method known in the art for introducing expression vectors into the cells. For example, transfection includes calcium phosphate or calcium chloride mediated transfection, lipofection, infection with a retroviral construct, and electroporation.

An alternative to the use of calcium indicator dyes is the use of the aequorin system. The aequorin system makes use of the protein apoaequorin, which binds to the lipophilic chromophore coelenterazine forming a combination of apoaequorin and coelenterazine that is known as aequorin. Apoaequorin has three calcium binding sites and, upon calcium binding, the apoaequorin portion of aequorin changes its conformation. This change in conformation causes coelenterazine to be oxidized into coelenteramide, CO₂, and a photon of blue light (466 nm). This photon can be detected with suitable instrumentation.

Since the gene encoding apoaequorin has been cloned (U.S. Patent No. 5,541,309; U.S. Patent No. 5,422,266; U.S. Patent No. 5,744,579; Inouye et al., 1985, Proc. Natl. Acad. Sci. USA 82:3154-3158; Prasher et al., 1985, Biochem. Biophys. Res. Comm. 126:1259-1268), apoaequorin can be recombinantly expressed in cells in which it is desired to measure the intracellular calcium concentration. Alternatively, existing cells that stably express recombinant apoaequorin can be used. Such cells derived from HEK293 cells and CHO-K1 cells are described in Button & Brownstein, 1993, Cell Calcium 14:663-671. For example, the HEK293/aeq17 cell line can be used as follows.

The HEK293/aeq17 cells are grown in Dulbecco's Modified Medium (DMEM, GIBCO-BRL, Gaithersburg, MD, USA) with 10% fetal bovine serum (heat inactivated), 1 mM sodium pyruvate, 500 µg/ml Geneticin, 100 µg/ml streptomycin, 100 units/ml penicillin. Expression vectors encoding the voltage-gated ion channel of interest as well as, optionally, the desired voltage-gated calcium channel subunits (α_{1A}, α_{1B}, α_{1C}, α_{1D}, α_{1E}, α_{1G}, α_{1H}, α_{1I}, α₂δ β₁, β₂, β₃, β₄, etc.) can be transfected into the HEK293/aeq17 cells by standard methods in order to express the desired voltage-gated ion channel subunits and voltage-gated calcium channel subunits in the HEK293/aeq17 cells. The cells are washed once with DMEM plus 0.1 % fetal bovine serum, and then charged for one hour at 37°C /5% CO₂ in DMEM containing 8 µM coelenterazine cp (Molecular Probes, Eugene, OR, USA) and 30 µM glutathione. The cells are then washed once with Versene (GIBCO-BRL, Gaithersburg, MD, USA), detached using Enzyme-free cellissociation buffer (GIBCO-BRL, Gaithersburg, MD, USA), diluted into ECB (Ham's F12 nutrient mixture (GIBCO-BRL) with 0.3 mM CaCl₂, 25 mM HEPES, pH7.3, 0.1% fetal bovine serum). The cell suspension is centrifuged at 500 x g for 5 min. The supernatant is removed, and the pellet is resuspended in 10 ml ECB. The cell density is determined by counting with a hemacytometer and adjusted to 500,000 cells/ml in ECB. The substances to be tested are diluted to the desired concentrations in ECB and aliquoted into the assay plates, preferably in triplicate, at 0.1 ml/well. The cell suspension is injected at 0.1 ml/well, read and integrated for a total of 400 readings using a luminometer (Luminoskan Ascent, Labsystems Oy, Helsinki, Finland). Alternatively, the cells may first be placed into the assay plates and then the substances added. Data are analyzed using the software GraphPad Prism Version 3.0 (GraphPad Software, Inc., San Diego, CA, USA).

It will be understood by those skilled in the art that the procedure outlined above is a general guide in which the various steps and variables can be modified somewhat to take into account the specific details of the particular assay that is desired to be run. For example, one could use semisynthetic coelenterazine (Shimomura, 1989, Biochem. J. 261:913-920; Shimomura et al., 1993, Cell Calcium 14:373-378); the time of incubation of the cells with coelenterazine can be varied somewhat; somewhat greater or lesser numbers of cells per well can be used; and so forth.

For reviews on the use of aequorin, see Créton et al., 1999, Microscopy Research and Technique 46:390-397; Brini et al., 1995, J. Biol. Chem. 270:9896-9903; Knight & Knight, 1995, Meth. Cell. Biol. 49:201-216. Also of interest may be U.S. Patent No. 5,714,666 which describes methods of measuring intracellular calcium in mammalian cells by the addition of coelenterazine co-factors to mammalian cells that express apoaequorin.

Another way to measure ion flow indirectly is to monitor changes in transcription that result from the activity of voltage-gated ion channels by the use of transcription based assays. Transcription-based assays involve the use of a reporter gene whose transcription is driven by an inducible promoter whose activity is regulated by a particular intracellular event such as, e.g., changes in intracellular calcium levels, that are caused by the activity of a voltage-gated ion channel. Transcription-based assays are reviewed in Rutter et al., 1998, Chemistry & Biology 5:R285-R290. Transcription-based assays of the present invention rely on the expression of reporter genes whose transcription is activated or repressed as a result of intracellular events that are caused by the interaction of a activator or inhibitor with a voltage-gated ion channel.

An extremely sensitive transcription-based assay is disclosed in Zlokarnik et al., 1998, Science 279:84-88 (Zlokarnik) and also in U.S. Patent No. 5,741,657. The assay disclosed in Zlokarnik and U.S. Patent No. 5,741,657 employs a plasmid encoding β-lactamase under the control of an inducible promoter. This plasmid is transfected into cells together with a plasmid encoding a receptor for which it is desired to identify agonists. The inducible promoter on the β-lactamase is chosen so that it responds to at least one intracellular signal that is generated when an agonist binds to the receptor. Thus, following such binding of agonist to receptor, the level of β-lactamase in the transfected cells increases. This increase in β-lactamase is measured by treating the cells with a cell-permeable dye that is a substrate for cleavage by β-lactamase. The dye contains two fluorescent moieties. In the intact dye, the two fluorescent moieties are physically linked, and thus close enough to one another that fluorescence resonance energy transfer (FRET) can take place between them. Following cleavage of the dye into two parts by β-lactamase, the two fluorescent moieties are located on different parts, and thus can diffuse apart. This increases the distance between the fluorescent moieties, thus decreasing the amount of FRET that can occur between them. It is this decrease in FRET that is measured in the assay.

The assay described in Zlokarnik and U.S. Patent No. 5,741,657 can be modified for use in the methods of the present invention by using an inducible promoter to drive β-lactamase where the promoter is activated by an intracellular signal generated by the opening or closing of a voltage-gated ion channel. Cells expressing a voltage-gated ion channel and the inducible promoter-driven β-lactamase are placed in the apparatus of the present invention, where the open or closed state of the voltage-gated ion channels can be controlled. The cells are exposed to the cell-permeable dye and then exposed to substances suspected of being activators or inhibitors of the voltage-gated ion channel. Those substances that cause a change in the open or closed state of the voltage-gated ion channel are identified by their effect on the inducible promoter-driven β-lactamase and thus on FRET. The inducible promoter-driven β-lactamase is engineered with a suitable promoter so that β-lactamase is induced when the substance is either an activator or an inhibitor, depending upon the nature of the assay.

The flow of ions through voltage-gated ion channels can also be measured by measuring changes in membrane potential via the use of fluorescent voltage sensitive dyes. The changes in membrane potential will depend on the ion channels in the cell membrane. The resultant membrane potential will depend on the net properties of all the channels and the change caused by inhibiting (through a substance that is an inhibitor or antagonist) or activating (through a substance that is an activator or an agonist) the voltage-gated ion channel of interest. One knowledgeable in cellular and membrane biophysics and electrophysiology will understand the directions of the changes in membrane potential since those changes depend on the ion channels present and the inhibition or activation of those channels by test substances. In many cases when using fluorescent voltage sensitive dyes, the experimental system can be calibrated by using known activators or inhibitors of the voltage-gated ion channel of interest.

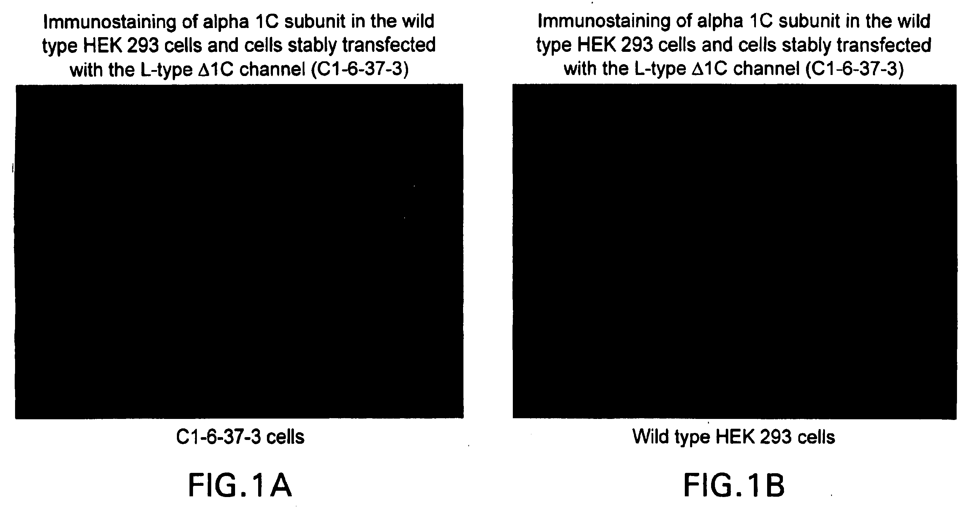

The present invention therefore includes assays that monitor changes in ion flow caused by activators or inhibitors of voltage-gated ion channels based upon FRET between a first and a second fluorescent dye where the first dye is bound to one side of the plasma membrane of a cell expressing a voltage-gated ion channel of interest and the second dye is free to move from one face of the membrane to the other face in response to changes in membrane potential. In certain embodiments, the first dye is impenetrable to the plasma membrane of the cells and is bound predominately to the extracellular surface of the plasma membrane. The second dye is trapped within the plasma membrane but is free to diffuse within the membrane. At normal (*i*.*e*., negative) resting potentials of the membrane, the second dye is bound predominately to the inner surface of the extracellular face of the plasma membrane, thus placing the second dye in close proximity to the first dye. This close proximity allows for the generation of a large amount of FRET between the two dyes. Following membrane depolarization, the second dye moves from the extracellular face of the membrane to the intracellular face, thus increasing the distance between the dyes. This increased distance results in a decrease in FRET, with a corresponding increase in fluorescent emission derived from the first dye and a corresponding decrease in the fluorescent emission from the second dye. See figure 1 of González & Tsien, 1997, Chemistry & Biology 4:269-277. See also González & Tsien, 1995, Biophys. J. 69:1272-1280 and U.S. Patent No. 5,661,035.

In certain embodiments, the first dye is a fluorescent lectin or a fluorescent phospholipid that acts as the fluorescent donor. Examples of such a first dye are: a coumarin-labeled phosphatidylethanolamine (*e*.*g*., N-(6-chloro-7-hydroxy-2-oxo-2H--1-benzopyran-3-carboxamidoacetyl)-dimyristoylphosphatidyl-ethanolamine) or N-(7-nitrobenz-2-oxa-1,3-diazol-4-yl)-dipalmitoylphosphatidylethanolamine); a fluorescently-labeled lectin (*e*.*g*., fluorescein-labeled wheat germ agglutinin). In certain embodiments, the second dye is an oxonol that acts as the fluorescent acceptor. Examples of such a second dye are: bis(1,3-dialkyl-2-thiobarbiturate)trimethineoxonols (*e*.*g*., bis(1,3-dihexyl-2-thiobarbiturate)trimethineoxonol) or pentamethineoxonol analogues (*e*.*g*., bis(1,3-dihexyl-2-thiobarbiturate)pentamethineoxonol; or bis(1,3-dibutyl-2-thiobarbiturate)pentamethineoxonol). See González & Tsien, 1997, Chemistry & Biology 4:269-277 for methods of synthesizing various dyes suitable for use in the present invention. In certain embodiments, the assay may comprise a natural carotenoid, *e*.*g*., astaxanthin, in order to reduce photodynamic damage due to singlet oxygen.

The use of such fluorescent dyes capable of moving from one face of the plasma membrane to the other is especially appropriate when the methods of the present invention are directed to inwardly rectifying potassium channels. Activation of inwardly rectifying potassium channels results in increased potassium current flow across the plasma membrane. This increased current flow results in a hyperpolarization of the cell membrane that can be detected by use of the technique described above since such hyperpolarization will result in greater FRET.

In particular embodiments of the present invention, cells are utilized that have been transfected with expression vectors comprising DNA that encodes a voltage-gated ion channel. Preferably, the cells do not naturally express corresponding voltage-gated ion channels. For example, if the expression vectors direct the expression of a voltage-gated calcium channel, the cells will not naturally express voltage-gated calcium channels. Alternatively, if the cells naturally express corresponding voltage-gated ion channels, those corresponding voltage-gated ion channels can be distinguished from the transfected voltage-gated ion channels in some manner, *e*.*g*., by the use of appropriate inhibitors, by manipulation of membrane potential. A preferred cell line for use in the present invention is the HEK293 cell line (ATCC 1573) since this cell line naturally expresses endogenous potassium channels, which may be beneficial for electrical field stimulation experiments with channels that cause membrane potential depolarization (*e*.*g*., sodium or calcium channels).

In a specific embodiment, the subject invention relates to a C1-6-37-3 cell and cell line. The C1-6-37-3 cell expresses the alpha1C calcium ion channel subunit and the Kir 2.3 inward rectifying potassium channel on its plasma membrane.

Cells are generally eukaryotic cells, preferably mammalian cells. The cells may be grown to the appropriate number on the substrates or they may be placed on the substrate and used without further growth. The cells may be attached to the substrate or, in those embodiments where the cells are placed or grown in wells, the cells may be suspension cells that are suspended in the fluid in the wells. Primary cells or established cell lines may be used.

Suitable cells for transfection with expression vectors that direct the expression of voltage-gated ion channels include but are not limited to cell lines of human, bovine, porcine, monkey and rodent origin. The cells may be adherent or non-adherent. Cells and cell lines which are suitable and which are widely available, include but are not limited to: L cells L-M(TK-) (ATCC CCL 1.3), L cells L-M (ATCC CCL 1.2), HEK293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), CPAE (ATCC CCL 209), Saos-2 (ATCC HTB-85), ARPE-19 human retinal pigment epithelium (ATCC CRL-2302), GH3 cells, T-REx-293 cells (Invitrogen, R710-07), T-REx-CHO cells (Invitrogen, R718-07) and primary cardiac myocytes.

A variety of voltage-gated ion channels may be used in the present invention. For example, voltage-gated sodium channels, voltage-gated potassium channels, and voltage-gated calcium channels are suitable.

In certain embodiments of the present invention, the cells used do not naturally express the voltage-gated ion channel of interest. Instead, DNA encoding the voltage-gated ion channel is transfected into cells in order to express the voltage-gated ion channel in the plasma membrane of the cells. DNA encoding voltage-gated ion channels can be obtained by methods well known in the art. For example, a cDNA fragment encoding a voltage-gated ion channel can be isolated from a suitable cDNA library by using the polymerase chain reaction (PCR) employing suitable primer pairs. The cDNA fragment encoding the voltage-gated ion channel can then be cloned into a suitable expression vector. Primer pairs can be selected based upon the known DNA sequence of the voltage-gated ion channel it is desired to obtain. Suitable cDNA libraries can be made from cellular or tissue sources known to contain mRNA encoding the voltage-gated ion channel.

One skilled in the art would know that for certain voltage-gated ion channels, it is desirable to transfect, and thereby express, more than one subunit in order to obtain a functional voltage-gated ion channel. For example, N-type calcium channels are composed of a multisubunit complex containing at least an α1B, an α2δ, and a β1 subunit. On the other hand, T-type calcium channels are functional with only a single subunit, *e*.*g*., α1G, α1H, or α1I. Common knowledge in the art of the subunit composition of a voltage-gated ion channel of interest will lead the skilled artisan to express the correct subunits in the transfected cells. U.S. Patent No. 5,851,824 provides sequences for the alpha.-1C/alpha-1D, alpha-2, β-1, and gamma.subunits

One skilled in the art could use published voltage-gated ion channel sequences to design PCR primers and published studies of voltage-gated ion channel expression to select the appropriate sources from which to make cDNA libraries in order to obtain DNA encoding the voltage-gated ion channels. The following publications may be of use in this regard:
U.S. Patent No. 5,876,958;
U.S. Patent No. 6,096,514;
U.S. Patent No. 6,090,623
Hondeghem, L.M., Katzung, B.G. (1984) Antiarrhythmic agents: the modulated receptor mechanism of action of sodium and calcium channel-blocking drugs. Annu-Rev-Pharmacol-Toxicol. 24:387-423.;
Zheng, W., Stoltefuss, J., Goldmann, S., and Triggle, D.J. (1992) Pharmacologic and radioligand binding studies of 1,4-dihydropyridines in rat cardiac and vasculr preparations: stereoselectivity and voltage dependence of antagonist and activator interactions. Mol. Pharmacol. 41(3):535-541.; and
Triggle, D.J., Hawthorn, M.H. and Zheng, W. (1988) Potential-dependent interactions of nitrendipine and related 1,4-dihydropyridines in functional smooth muscle preparations. J. Cardiovasc. Pharmacol., 12(Suppl.4):s91-s93.

The following table provides a list of known ion channels and information concerning each:

**TABLE 1**

| Some ion channel genes of interest for ion flux experiments | | | | |
|---|---|---|---|---|
| Symbol | Full Name | Cytogenetic Location | MIM Number | PubMed ID |
| SCN1 | symbol withdrawn, see SCN1A | | | |
| SCN1A | sodium channel, voltage-gated, type I, alpha polypeptide | 2q24 | 182389 | 8062593 |
| SCN1B | sodium channel, voltage-gated, type I, beta polypeptide | 19 | 600235 | 8394762 |
| SCN2A1 | sodium channel, voltage-gated, type II, alpha 1 polypeptide | 2q22-q23 | 182390 | 1317301 |
| SCN2A2 | sodium channel, voltage-gated, type II, alpha 2 polypeptide | 2q23-q24 | 601219 | 1317301 |
| SCN2A | symbol withdrawn, see SCN2A1 | - | | |
| SCN2B | sodium channel, voltage-gated, type II, beta polypeptide | 11q22-qter | 601327 | 10198179 |
| SCN3A | sodium channel, voltage-gated, type III, alpha polypeptide | 2q24 | 182391 | 9589372 |
| SCN4A | sodium channel, voltage-gated, type IV, alpha polypeptide | 17q23-q25.3 | 603967 | 1654742 |
| SCN4B | sodium channel, voltage-gated, type IV, beta polypeptide | reserved | | |
| SCN5A | sodium channel, voltage-gated, type V, alpha polypeptide (long (electrocardiographic) QT syndrome 3) | 3p21 | 600163 | |
| SCN6A | sodium channel, voltage-gated, type VI, alpha polypeptide | 2q21-q23 | 182392 | 10198179 |
| SCN7A | symbol withdrawn, see SCN6A | - | | |
| SCN8A | sodium channel, voltage gated, type VIII, alpha polypeptide | 12q13.1 | 600702 | 7670495 |
| SCN9A | sodium channel, voltage-gated, type IX, alpha polypeptide | 2q24 | 603415 | 7720699 |
| SCN10A | sodium channel, voltage-gated, type X, alpha polypeptide | 3p21-p22 | 604427 | 9839820 |
| SCN11A | sodium channel, voltage-gated, type XI, alpha polypeptide | 3p21-p24 | 604385 | 10444332 |
| SCN12A | sodium channel, voltage-gated, type XII, alpha polypeptide | 3p23-p21.3 | | 10623608 |
| SCNN1 | symbol withdrawn, see SCNN1A | - | | |
| SCNN1A | sodium channel, nonvoltage-gated 1 alpha | 12p13 | 600228 | 7896277 |
| SCNN1B | sodium channel, nonvoltage-gated I, beta (Liddle syndrome) | 16p12.2-p12.1 | | 600760 |
| SCNN1D | sodium channel, nonvoltage-gated 1, delta | 1p36.3-p36.2 | 601328 | 8661065 |
| SCNN1G | sodium channel, nonvoltage-gated 1, gamma | 16p12 | 600761 | 7490094 |
| CACNAIA | calcium channel, voltage-dependent, P/Q type, alpha 1A subunit | 19p13 | 601011 | 8825650 |
| CACNA1B | calcium channel, voltage-dependent, L type, alpha 1B subunit | 9q34 | 601012 | 8825650 |
| CACNA1C | calcium channel, voltage-dependent, L type, alpha 1C subunit | 12pter-p13.2 | 114205 | 1650913 |
| CACNA1D | calcium channel, voltage-dependent, L type, alpha 1D subunit | 3p14.3 | 114206 | 1664412 |
| CACNA1E | calcium channel, voltage-dependent, alpha IE subunit | 1q25-q31 | 601013 | 8388125 |
| CACNA1F | calcium channel, voltage-dependent, alpha IF subunit | Xp11.23-p11.22 | 300110 | 9344658 |
| CACNA1G | calcium channel, voltage-dependent, alpha 1G subunit | 17q22 | 604065 | 9495342 |
| CACNA1H | calcium channel, voltage-dependent, alpha IH subunit | 16p13.3 | | 9670923 |
| CACNA1I | calcium channel, voltage-dependent, alpha 1I subunit | 22q12.3-13.2 | | 10454147 |
| CACNA1S | calcium channel, voltage-dependent, L type, alpha 1S subunit | 1q31-q32 | 114208 | 7916735 |
| CACNA2 | symbol withdrawn, see CACNA2D1 | - | | |
| CACNA2D1 | calcium channel, voltage-dependent, alpha 2/delta subunit 1 | 7q21-q22 | 114204 | 8188232 |
| CACNA2D2 | calcium channel, voltage-dependent, alpha 2/delta subunit 2 | reserved | | |
| CACNB1 | calcium channel, voltage-dependent, beta 1 subunit | 17q21-q22 | 114207 | 8381767 |
| CACNB2 | calcium channel, voltage-dependent, beta 2 subunit | 10p12 | 600003 | 9254841 |
| CACNB3 | calcium channel, voltage-dependent, beta 3 subunit | 12q13 | 601958 | 8119293 |
| CACNB4 | calcium channel, voltage-dependent, beta 4 subunit | 2q22-q31 | 601949 | 9628818 |
| CACNG1 | calcium channel, voltage-dependent, gamma subunit 1 | 17q24 | 114209 | 8395940 |
| CACNG2 | calcium channel, voltage-dependent, gamma subunit 2 | reserved | 602911 | |
| CACNG3 | calcium channel, voltage-dependent, gamma subunit 3 | reserved | | |
| CACNG4 | calcium channel, voltage-dependent, gamma subunit 4 | 17q24 | | 10613843 |
| CACNG5 | calcium channel, voltage-dependent, gamma subunit 5 | 17q24 | | 10613843 |
| CACNG6 | calcium channel, voltage-dependent, gamma subunit 6 | 19q13.4 | | 11170751 |
| CACNG7 | calcium channel, voltage-dependent, gamma subunit 7 | 19q13.4 | | 11170751 |
| CACNG8 | calcium channel, voltage-dependent, gamma subunit 8 | 19q13.4 | | 11170751 |
| KCNA1 | potassium voltage-gated channel, shaker-related subfamily, member I (episodic ataxia with myokymia) | 12p13 | 176260 | 1349297 |
| KCNA1B | literature alias, see KCNAB1 | - | | |
| KCNA2 | potassium voltage-gated channel, shaker- related subfamily, member 2 | 12 | 176262 | |
| KCNA2B | literature alias, see KCNAB2 | - | | |
| KCNA3 | potassium voltage-gated channel, shaker- related subfamily, member 3 | 1p13.3 or 13 | 176263 | 2251283 |
| KCNA3B | literature alias, see KCNAB3 | - | | |
| KCNA4 | potassium voltage-gated channel, shaker-related subfamily, member 4 | 11p14 | 176266 | 2263489 |
| KCNA4L | potassium voltage-gated channel, shaker-related subfamily, member 4-like | 11q14 | | 8449523 |
| KCNA5 | potassium voltage-gated channel, shaker-related subfamily, member 5 | 12 | 176267 | |
| KCNA6 | potassium voltage-gated channel, shaker-related subfamily, member 6 | reserved | 176257 | |
| KCNA7 | potassium voltage-gated channel, shaker-related subfamily, member 7 | 19 | 176268 | |
| KCNA8 | literature alias, see KCNQ1 | - | | |
| KCNA9 | symbol withdrawn, see KCNQ1 | - | | |
| KCNA10 | potassium voltage-gated channel, shaker-related subfamily, member 10 | reserved | 602420 | |
| KCNAB1 | potassium voltage-gated channel, shaker-related subfamily, beta member 1 | 3q26.1 | 601141 | 8838324 |
| KCNAB2 | potassium voltage-gated channel, shaker-related subfamily, beta member 2 | 1p36.3 | 601142 | 8838324 |
| KCNAB3 | potassium voltage-gated channel, shaker-related subfamily, beta member 3 | 17p13.1 | 604111 | 9857044 |
| KCNB1 | potassium voltage-gated channel, Shab-related subfamily, member I | 20q 13.2 | 600397 | 7774931 |
| KCNB2 | potassium voltage-gated channel, Shab-related subfamily, member 2 | 8 | | 9612272 |
| KCNC1 | potassium voltage-gated channel, Shaw-related subfamily, member 1 | 11p15 | 176258 | 8449507 |
| KCNC2 | potassium voltage-gated channel, Shaw-related subfamily, member 2 | 12 and 19q 13.4 | 176256 | 8111118 |
| KCNC3 | potassium voltage-gated channel, Shaw-related subfamily, member 3 | 19 | 176264 | 1740329 |
| KCNC4 | potassium voltage-gated channel, Shaw-related subfamily, member 4 | 1p21 | 176265 | 1920536 |
| KCND1 | potassium voltage-gated channel, Shal-related subfamily, member I | Xp11.23-p11.3 | 300281 | 10729221 |
| KCND2 | potassium voltage-gated channel, Shal-related subfamily, member 2 | 7q31-32 | 605410 | 10551270 |
| KCND3 | potassium voltage-gated channel, Shal-related subfamily, member 3 | 1p13.2 | 605411 | 10942109 |
| KCNE1 | potassium voltage-gated channel, Isk-related family, member I | 21q22.1-q22.2 | 176261 | 8432548 |
| KCNE1L | potassium voltage-gated channel, Isk-related family, member 1-like | Xq22.3 | 300328 | 10493825 |
| KCNE2 | potassium voltage-gated channel, Isk-related family, member 2 | 21q22.1 | 603796 | 10219239 |
| KCNE3 | potassium voltage-gated channel, Isk-related family, member 3 | reserved | 604433 | 10219239 |
| KCNE4 | potassium voltage-gated channel, Isk-related family, member 4 | reserved | | 10219239 |
| KCNF1 | potassium voltage-gated channel, subfamily F, member 1 | 2p25 | 603787 | 9434767 |
| KCNF2 | literature alias, see KCNG2 | - | | |
| KCNF | symbol withdrawn, see KCNF1 | - | | |
| KCNG1 | potassium voltage-gated channel, subfamily G, member 1 | 20q13 | 603788 | 9434767 |
| KCNG2 | potassium voltage-gated channel, subfamily G, member 2 | 18q22-18q23 | 605696 | 10551266 |
| KCNG | symbol withdrawn, see KCNG1 | - | | |
| KCNH1 | potassium voltage-gated channel, subfamily H (eag-related), member 1 | 1q32-41 | 603305 | 9738473 |
| KCNH2 | potassium voltage-gated channel, subfamily H (eag-related), member 2 | 7q35-q36 | 152427 | 7842012 |
| KCNH3 | potassium voltage-gated channel, subfamily H (eag-related), member 3 | 12q13 | 604527 | 10455180 |
| KCNH4 | potassium voltage-gated channel, subfamily H (eag-related), member 4 | reserved | 604528 | 10455180 |
| KCNH5 | potassium voltage-gated channel, subfamily H (eag-related), member 5 | 14 | 605716 | 9738473 |
| KCNIP1 | Kv channel interacting protein 1 | reserved | | 10676964 |
| KCNIP2 | Kv channel-interacting protein 2 | 10 | | 10676964 |
| KCNIP3 | literature alias, see CSEN | - | | |
| KCNJ1 | potassium inwardly-rectifying channel, subfamily J, member 1 | 11q24 | 600359 | 7680431 |
| KCNJ2 | potassium inwardly-rectifying channel, subfamily J, member 2 | 17q23.1-q24.2 | 600681 | 7696590 |
| KCNJ3 | potassium inwardly-rectifying channel, subfamily J, member 3 | 2q24.1 | 601534 | 8088798 |
| KCNJ4 | potassium inwardly-rectifying channel, subfamily J, member 4 | 22q13.1 | 600504 | 8016146 |
| KCNJ5 | potassium inwardly-rectifying channel, subfamily J, member 5 | 11q24 | 600734 | |
| KCNJ6 | potassium inwardly-rectifying channel, subfamily J, member 6 | 21q22.1 | 600877 | 7796919 |
| KCNJ7 | symbol withdrawn, see KCNJ6 | - | | |
| KCNJ8 | potassium inwardly-rectifying channel, subfamily J, member 8 | 12p11.23 | 600935 | 8595887 |
| KCNJ9 | potassium inwardly-rectifying channel, subfamily J, member 9 | 1q21-1q23 | 600932 | 8575783 |
| KCNJ10 | potassium inwardly-rectifying channel, subfamily J, member 10 | 1q | 602208 | 9367690 |
| KCNJ11 | potassium inwardly-rectifying channel, subfamily J, member 11 | 11p15.1 | 600937 | 7502040 |
| KCNJ12 | potassium inwardly-rectifying channel, subfamily J, member 12 | 17p11.1 | 602323 | 7859381 |
| KCNJ13 | potassium inwardly-rectifying channel, subfamily J, member 13 | 2q37 | 603208 | 9878260 |
| KCNJ14 | potassium inwardly-rectifying channel, subfamily J, member 14 | 19q13 | 603953 | 9592090 |
| KCNJ15 | potassium inwardly-rectifying channel, subfamily J, member 15 | 21q22.2 | 602106 | 9299242 |
| KCNJ16 | potassium inwardly-rectifying channel, subfamily J, member 16 | 17q23.1-q24.2 | 605722 | 11240146 |
| KCNJN1 | channel, subfamily J, inhibitor 1 | 17p11.2-p11.1 | 602604 | 8647284 |
| KCNK1 | potassium channel, subfamily K, member 1 (TWIK-1) | 1q42-q43 | 601745 | 8661042 |
| KCNK2 | potassium channel, subfamily K, member 2 (TREK-1) | 1q41 | 603219 | 9721223 |
| KCNK3 | potassium channel, subfamily K, member 3 (TASK-1) | 2p23 | 603220 | 9312005 |
| KCNK4 | potassium inwardly-rectifying channel, subfamily K, member 4 | 11q13 | 605720 | 10767409 |
| KCNK5 | potassium channel, subfamily K, member 5 (TASK-2) | 6p21 | 603493 | 9812978 |
| KCNK6 | potassium channel, subfamily K, member 6 (TWIK-2) | 19q13.1 | 603939 | 10075682 |
| KCNK7 | potassium channel, subfamily K, member 7 | 11q13 | 603940 | 10206991 |
| KCNK9 | potassium channel, subfamily K, member 9 (TASK-3) | 8 | 605874 | 10734076 |
| KCNK10 | potassium channel, subfamily K, member 10 | reserved | 605873 | |
| KCNK12 | potassium channel, subfamily K, member 12 | 2p22-2p21 | | |
| KCNK13 | potassium channel, subfamily K, member 13 | 14q24.1-14q24.3 | | 11060316 |
| KCNK14 | potassium channel, subfamily K, member 14 | 2p22-2p21 | | 11060316 |
| KCNK15 | potassium channel, subfamily K, member 15 | reserved | | |
| KCNMA1 | potassium large conductance calcium-activated channel, subfamily M, alpha member 1 | 10 | 600150 | 7987297 |
| KCNMB1 | potassium large conductance calcium-activated channel, subfamily M, beta member 1 | 5q34 | 603951 | 8799178 |
| KCNMB2 | symbol withdrawn, see KCNMB3 | - | | |
| KCNMB2 | potassium large conductance calcium-activated channel, subfamily M, beta member 2 | reserved | 605214 | 10097176 |
| KCNMB2L | symbol withdrawn, see KCNMB3L | - | | |
| KCNMB3 | potassium large conductance calcium-activated channel, subfamily M beta member 3 | 3q26.3-q27 | 605222 | 10585773 |
| KCNMB3L | potassium large conductance calcium-activated channel, subfamily M, beta member 3-like | 22q11 | | 10585773 |
| KCNMB4 | potassium large conductance calcium-activated channel, subfamily M, beta member 4 | reserved | 605223 | |
| KCNMBL | symbol withdrawn, see KCNMB3 | - | | |
| KCNMBLP | symbol withdrawn, see KCNMB3L | - | | |
| KCNN1 | potassium intermediate/small conductance calcium-activated channel, subfamily N, member I | 19p13.1 | 602982 | 8781233 |
| KCNN2 | potassium intermediate/small conductance calcium-activated channel, subfamily N, member 2 | reserved | 605879 | |
| KCNN3 | potassium intermediate/small conductance calcium-activated channel, subfamily N, member 3 | 22q11-q13.1 | 602983 | 9491810 |
| KCNN4 | potassium intermediate/small conductance calcium-activated channel, subfamily N, member 4 | 19q13.2 | 602754 | 9380751 |
| KCNQ1 | potassium voltage-gated channel, KQT-like subfamily, member 1 | 11p15.5 | 192500 | 8528244 |
| KCNQ1OT1 | KCNQ1 overlapping transcript 1 | 11p15.5 | 604115 | 10220444 |
| KCNQ2 | potassium voltage-gated channel, KQT-like subfamily, member 2 | 20q13.3-2 20q13.3 | 121200 | 9425895 |
| KCNQ3 | potassium voltage-gated channel, KQT-like subfamily, member 3 | 8q24 | 121201 | 9425900 |
| KCNQ4 | potassium voltage-gated channel, KQT-like subfamily, member 4 | 1p34 | 603537 | 10025409 |
| KCNQ5 | potassium voltage-gated channel, KQT-like subfamily, member 5 | 6q14 | | 10787416 |
| KCNS1 | potassium voltage-gated channel, delayed-rectifier, subfamily S, member 1 | reserved | 602905 | 9305895 |
| KCNS2 | potassium voltage-gated channel, delayed-rectifier, subfamily S, member 2 | 8q22 | 602906 | 9305895 |
| KCNS3 | potassium voltage-gated channel, delayed-rectifier, subfamily S, member 3 | reserved | 603888 | 10484328 |

PCR reactions can be carried out with a variety of thermostable enzymes including but not limited to AmpliTaq, AmpliTaq Gold, or Vent polymerase. For AmpliTaq, reactions can be carried out in 10 mM Tris-Cl, pH 8.3, 2.0 mM MgCl₂, 200 µM of each dNTP, 50 mM KCI, 0.2 µM of each primer, 10 ng of DNA template, 0.05 units/µl of AmpliTaq. The reactions are heated at 95°C for 3 minutes and then cycled 35 times using suitable cycling parameters, including, but not limited to, 95°C, 20 seconds, 62°C, 20 seconds, 72°C, 3 minutes. In addition to these conditions, a variety of suitable PCR protocols can be found in PCR Primer, A Laboratory Manual, edited by C.W. Dieffenbach and G.S. Dveksler, 1995, Cold Spring Harbor Laboratory Press; or PCR Protocols: A Guide to Methods and Applications, Michael et al., eds., 1990, Academic Press.

It is desirable to sequence the DNA encoding voltage-gated ion channels obtained by the herein-described methods, in order to verify that the desired voltage-gated ion channel has in fact been obtained and that no unexpected changes have been introduced into its sequence by the PCR reactions. The DNA can be cloned into suitable cloning vectors or expression vectors, *e.g.,* the mammalian expression vector pcDNA3.1 (Invitrogen, San Diego, CA) or other expression vectors known in the art or described herein.

A variety of expression vectors can be used to recombinantly express DNA encoding voltage-gated ion channels for use in the present invention. Commercially available expression vectors which are suitable include, but are not limited to, pMC1neo (Stratagene), pSG5 (Stratagene), pcDNAI and pcDNAIamp, pcDNA3, pcDNA3.1, pCR3.1 (Invitrogen, San Diego, CA), EBO-pSV2-neo (ATCC 37593), pBPV-1(8-2) (ATCC 37110), pdBPV-MMTneo(342-12) (ATCC 37224), pRSVgpt (ATCC 37199), pRSVneo (ATCC 37198), pCI.neo (Promega), pTRE (Clontech, Palo Alto, CA), pV1Jneo, pIRESneo (Clontech, Palo Alto, CA), pCEP4 (Invitrogen, San Diego, CA), pSC11, and pSV2-dhfr (ATCC 37146). The choice of vector will depend upon cell type in which it is desired to express the voltage-gated ion channels, as well as on the level of expression desired, and the like.

The expression vectors can be used to transiently express or stably express the voltage-gated ion channels. The transient expression or stable expression of transfected DNA is well known in the art. See, *e*.*g*., Ausubel et al., 1995, "Introduction of DNA into mammalian cells," in Current Protocols in Molecular Biology, sections 9.5.1-9.5.6 (John Wiley & Sons, Inc.).

As an alternative to the above-described PCR methods, cDNA clones encoding ion channels can be isolated from cDNA libraries using as a probe oligonucleotides specific for the desired voltage-gated ion channels and methods well known in the art for screening cDNA libraries with oligonucleotide probes. Such methods are described in, *e*.*g*., Sambrook et al., 1989, Molecular Cloning: A Laboratory Manual; Cold Spring Harbor Laboratory, Cold Spring Harbor, New York; Glover, D.M. (ed.), 1985, DNA Cloning: A Practical Approach, MRL Press, Ltd., Oxford, U.K., Vol. I, II. Oligonucleotides that are specific for particular voltage-gated ion channels and that can be used to screen cDNA libraries can be readily designed based upon the known DNA sequences of the voltage-gated ion channels and can be synthesized by methods well-known in the art.

### EXAMPLE 1

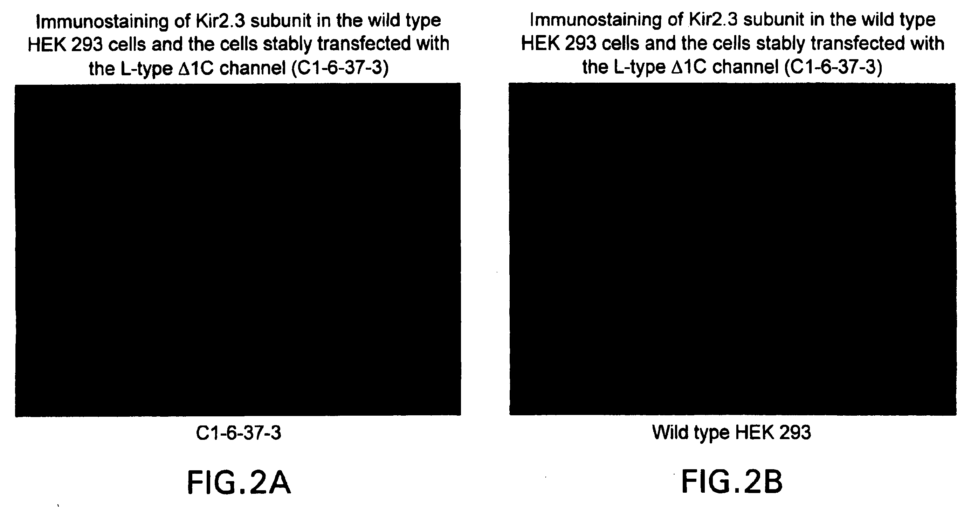

Immunofluorescence staining was all performed at room temperature. Cells were washed three times with Dulbecco's phosphate buffered saline (D-PBS) and then fixed with 4% paraformldehyde for 30 min. After three washes with D-PBS, the cells were blocked and permeabilized with TBS (10 mM Tris-HCl, pH 7.5, and 150 mM NaCl) containing 4% nonfat dry milk and 0.1 % Triton X-100 for 1 hr, and incubated with the affinity purified polyclonal antibodies against human alpha 1C or kir2.3 for 1 hr. Then the cells were washed three times with TBS and incubated with the secondary antibody (Cy3-conjugated anti-rabbit IgG, at 1:250, Jackson ImmunoResearch, PA) for 1 hr. The cells were finally washed with D-PBS three times and viewed under indirect immunofluorescence on a Zeiss Axioskop microscope. Figures 1 and 2 show that cells were successfully transfected and expressing calcium and potassium channels, respectively, on their plasma membranes.

### EXAMPLE 2

Hek 293 cells were stably transfected with the alpha 1C subunit of the L-type Calcium ion channel and Kir 2.3 inward K⁺ rectifying channel (C1-6-37-3 cells). Calcium influx into the cells was measured in a FLIPR™ (Molecular Devices, CA). The C1-6-37-3 cells were seeded into black 96 well plates with clear bottoms coated with poly-D-lysine at density of 50000 cells/well and cultured overnight. Next day the cells were washed twice with assay buffer containing 137 mM NaCl; 0.34 mM Na₂HPO₄; 4.2 mM NaHCO₃; 0.44 mM KH₂PO₄; 0.41 mM MgSO₄; 0.49 mM MgCl₂; 20 mM HEPES; 5.5 mM D-glucose and 0.1% BSA and incubated with Fluo-3AM (final concentration 4 µM, Molecular probe) for 1 hr at 37°C, 5% CO₂ and 95% O₂. After cells were washed four times either with resting condition (5.8 K⁺) or depolarized condition (30 mM K⁺), the cell plate was placed into the FLIPR™ to monitor cell fluorescence (λ_{EX}=488 nm, λ_{EM}=540 nm) before and after the addition of calcium blockers and agonists (final 85.8 mM K⁺).

Cellular membrane potentials were measured using an Axopatch 200B patch amplifier (Axon Instruments Inc., Foster City, CA) in current clamp more using the "perforated patch" clamp method (Horn and Korn). The patch pipette contained (in mM): 120 KMeS04, 20 KCl, 9 Mg2Cl, 10 HEPES, Nystatin 200 µg/ml, pH 7.3. The bath solution contained (in mM): 140 NaCl, 1.2 Mg2Cl, 10 HEPES, 1.3 Ca2Cl, 21 D-glucose, pH 7.4. Standard electrophysiological methods were employed. Changes in extracellular potassium were made by additions of a concentrated stock to the standard bath solution to the appropriate dilution.

### Results:

Table 2 shows the membrane potential of the C1-6-37-3 cells recorded at various extracellular potassium concentrations. This experiment confirms that changes in potassium alter the membrane potential of these cells approximately as predicted by the Nernst equation.

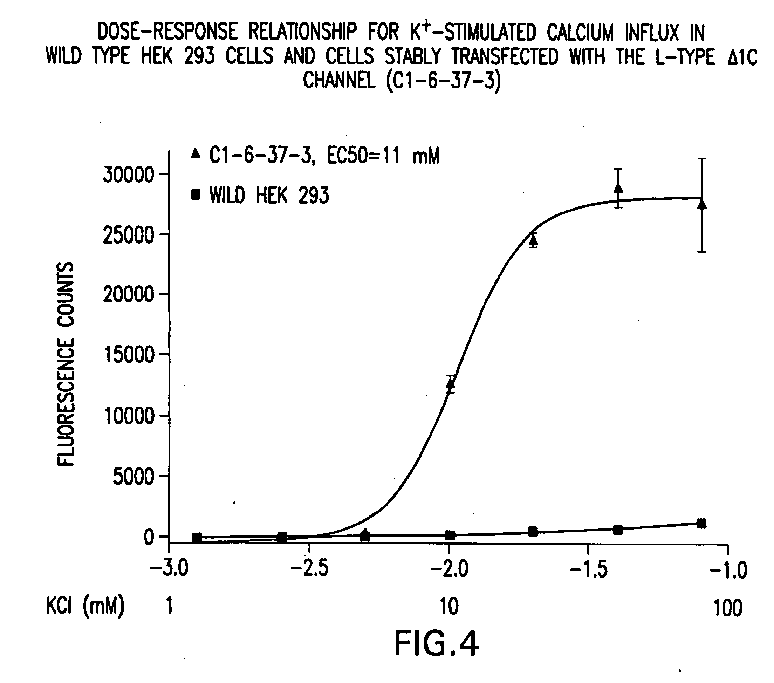

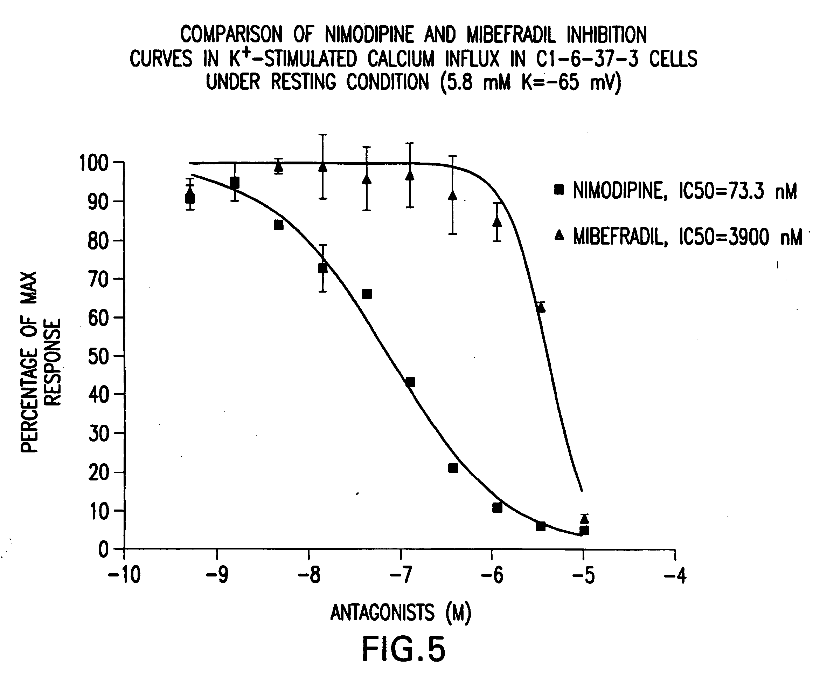

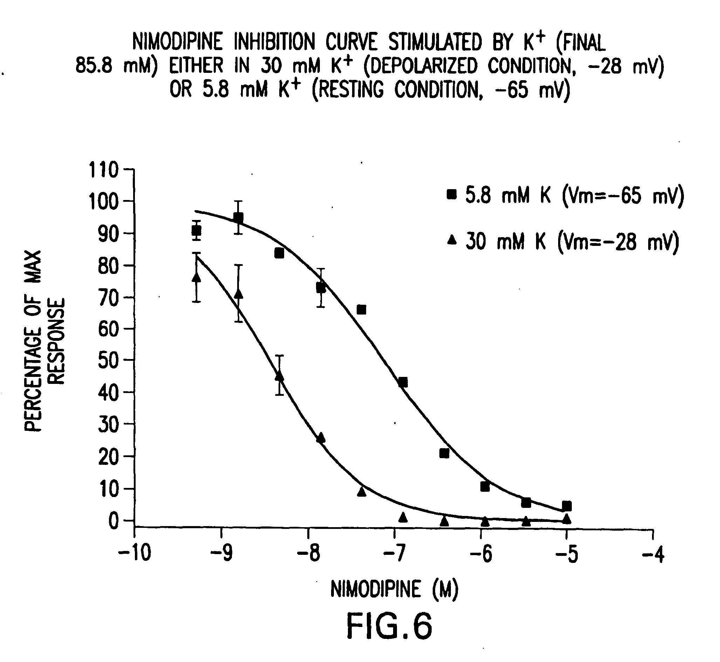

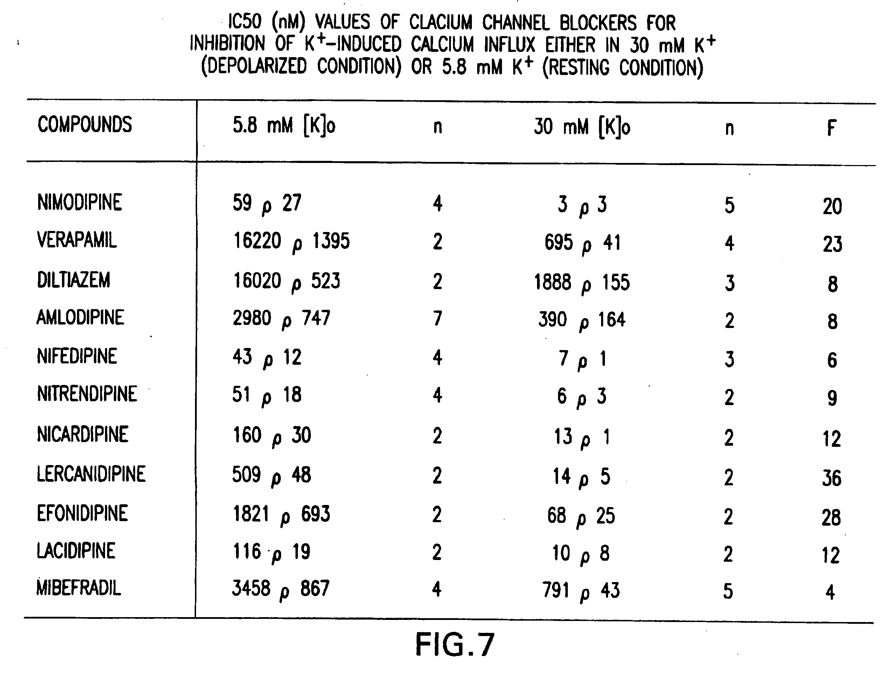

Figure 4 shows that calcium influx into fluo-3 loaded cells in response to increasing potassium concentration was concentration dependent and possessed an EC₅₀ of 11 mM K⁺. The potency of the inhibitory effect of nimodipine and other calcium channel antagonists on calcium influx through the α1C channel was shown to depend on membrane potential (table 3, Figures 5-7). Preincubation of cells with 30 mM K+ (Vm =-28 mV) increased the potency of nimodipine to block calcium influx compared to the preincubation of these cells with 5.8 mM K⁺ (Vm = -65 mV). This assay captures the state-dependent interactions of 1,4-dihydropyridines that have been identified previously.

**Table 2. Membrane potential of C 1-6-37-3 cell line recorded in various potassium concentrations using Nystatin perforated patch**

| [K]ₒᵤₜ mM | Resting potential | n |
|---|---|---|
| 0.4 | -99.3 ± 10.6 | 6 |
| 4.0 | -73 ± 0.7 | 6 |
| 5.8 | -64.7 ± 2.6 | 7 |
| 30 | -27.6 ± 2.4 | 7 |
| 80 | 7.5 ± 7.1 | 7 |

| | | |
|---|---|---|
| Values are the mean ± | | |

**Table 3. IC50 (nM) values of calcium channel antagonists for inhibition of K⁺-induced calcium influx either in 30 mM K⁺ (depolarized condition) or 5.8 mM K⁺ (resting condition).**

| Antagonists | 5.8 mM [K]o | n | 30 mM [K]o | n | F |
|---|---|---|---|---|---|
| Nimodipine | 59 ± 27 | 4 | 3 ± 3 | 5 | 21 |
| Nifedipine | 43 ± 12 | 4 | 7 ± 1 | 3 | 7 |
| Nitrendipine | 51 ± 18 | 4 | 6 ± 3 | 2 | 8 |
| Mibefradil | 3458 ± 867 | 4 | 791 ± 43 | 5 | 4 |

| | | | | | |
|---|---|---|---|---|---|
| Values are the mean ± S.D. F indicates the ratio of the IC50 values of 5.8 mM K⁺ and 30 mM K⁺. | | | | | |

### EXAMPLE 3

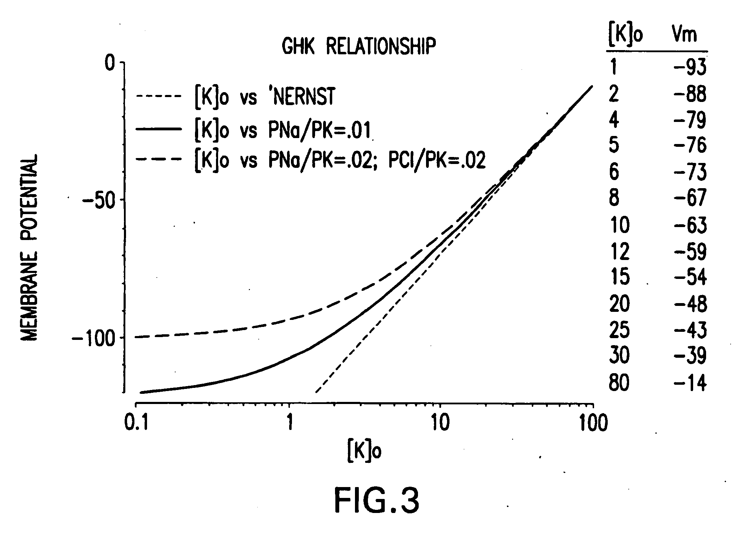

Cellular membrane potentials were measured using an Axopatch 200B patch amplifier (Axon Instruments Inc., Foster City, CA) in current clamp more using the 'perforated patch' clamp method (Horn and Korn). The patch pipette contained (in mM): 120 KMeS04, 20 KCI, 9 Mg2Cl, 10 HEPES, Nystatin 200 µg/ml, pH7.3. The bath solution contained (in mM): 140 NaCl, 1.2 Mg2Cl, 10 HEPES, 1.3 Ca2Cl, 21 D-glucose, pH7.4. Standard electrophysiological methods were employed. Changes in extracellular potassium were made by additions of a concentrated stock to the standard bath solution to the appropriate dilution. Figure 3 shows the relationship between extracellular potassium ([K]o) and cell membrane potential. Three situations are shown. One is the prediction of the Nernst equation for a perfectly K-selective membrane. The other curves show the effects of partial permeability by other ions, Na⁺ and/or Cl⁻. Membrane potential can be set in a non-voltage clamped cell by adjusting external potassium. A cell line expressing an inward rectifier K channel (Kir2.3) to set the resting membrane potential will permit control of membrane resting potential by extracellular potassium.

## Claims

1. A eukaryotic cell transformed to express a functional voltage-gated calcium ion channel and a functional inward rectifier potassium channel.

2. A cell according to claim 1 in which the channels are expressed on the plasma membrane of the cell.

3. A cell according to claim 1 or 2 where the voltage-gated calcium ion channel is an L-type calcium channel.

4. A cell according to claim 3 where the L-type calcium channel comprises an α1C subunit.

5. A cell according to claim 4 where the L-type calcium channel is a complex comprising the α1C, α2-δ and β2a subunits.

6. A cell according to any preceding claim where the inward rectifier potassium channel is a Kir2.3 channel.

7. A cell according to any previous claim which is selected from: L cells L-M(TK-) (ATCC CCL 1.3), L cells L-M (ATCC CCL 1.2), HEK293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa. (ATCC CCL 2), C127I (ATCC CRL 1616), BS-C-1 (ATCC CCL 26), MRC-5 (ATCC CCL 171), CPAE (ATCC CCL 209), Saos-2 (ATCC HTB-85), ARPE-19 human retinal pigment epithelium (ATCC CRL-2302), GH3 cells, TREx-292 cells, T-REx-CHO cells, and primary cardiac myocytes.

8. A cell according to claim 7 which is a HEK293 cell.

9. A method for testing a compound for activity as an agonist or antagonist of a calcium channel, comprising the steps of:
(a) contacting a cell as defined in any preceding claim with a solution having a potassium concentration where the membrane potential of the cell is modulated without fully depolarizing the cell;
(b) simultaneous to or subsequent to step (a), contacting the cell with (i) a substance of interest and (ii) an ion or molecule capable of entering the cell through a functional calcium channel;
(c) depolarizing the cell membrane of the cell;
(d) detecting the channel mediated ion flux into the cell; and
(e) comparing the ion flux thus detected from step (d) to an ion flux produced in a control experiment, wherein the control experiment comprises subjecting a separate cell to the steps (a), (b)(ii), (c) and (d), but not step (b)(i);
where a difference in ion flux detected in step (d) and the control experiment indicates that the substance of interest is an agonist or antagonist of a calcium channel.

10. A method according to claim 9 for identifying state-dependent antagonists of a voltage-gated calcium ion channel comprising:
(a) providing a divided tissue culture plate comprising individual compartments, where at least two of the individual compartments containing a plurality of living eukaryotic cells as defined in any one of claims 1 to 8, the cytoplasm of the cells comprising an ion-sensitive fluorescent indicator compound;
(b) adjusting the membrane potential of the cells by altering extracellular potassium concentration in at least one of the compartments containing the cells;
(c) adding a substance of interest to at least one of the individual compartments containing the cells;
(d) depolarizing the cells in the at least two compartments containing cells, wherein at least one compartment is subjected to step (c), test group, and at least one compartment is not subjected to step (c), control group;
(e) detecting the ion flux into the cells of step (d); and
(f) comparing the ion flux into the cells of the test group with the cells of the control group;
where if the value of ion flux in the test group cells is lower than the control group cells, the substance is an antagonist of the voltage-gated calcium ion channel.

11. The method of claim 9 further comprising comparing ion flux in the test group with that in a second test group, the second test group comprising cells subjected to steps (b) and (c), but whose membrane potentials have been adjusted to a value different than that of the test group cells;
where if the value of ion flux in the test group cells is different than the value of ion flux in the second test group cells, then the substance possesses a state-dependent potency on the voltage-gated calcium ion channel.

12. The method of claim 10 or 11, wherein the divided tissue culture plate is a multiwell tissue culture plate comprising at least two wells.

13. The method of claim 12, wherein the multiwell tissue culture plate comprises 12, 24, 96, 384, 1,536, or 3,456 wells.

14. The method of any one of claims 9 to 13, wherein at least 10 substances are tested in a 24 hour period.

15. The method of any one of claims 10 to 14 wherein the fluorescent indicator compound is selected from the group consisting of fluo-3, fura-2, fluo-4, fluo-5, calcium green-1, Oregon green, 488 BAPTA, SNARF-1, and indo-1.

16. The method of any one of claims 10 to 15, wherein the detecting step (e) employs a fluorescence or luminescence indicator device.

17. The method of any one of claims 10 to 16, wherein the detecting step (e) employs a Fluorimetric Imaging Plate Reader (FLIPR) or Voltage/Ion Probe Reader (VIPR) device.

## Patentansprüche

1. Eine eukaryotische Zelle, transformiert, um einen funktionellen spannungsgesteuerten Calciumionenkanal und einen funktionellen einwärts gerichteten Gleichrichter-Kaliumkanal zu exprimieren.

2. Eine Zelle gemäß Anspruch 1, wobei die Kanäle auf der Plasmamembran der Zelle exprimiert werden.

3. Eine Zelle gemäß Anspruch 1 oder 2, wobei der spannungsgesteuerte Calciumionenkanal ein L-Typ-Calciumkanal ist.

4. Eine Zelle gemäß Anspruch 3, wobei der L-Typ-Calciumkanal eine α1C-Untereinheit enthält.

5. Eine Zelle gemäß Anspruch 4, wobei der L-Typ-Calciumkanal ein Komplex ist, der die α1C-, α2-δ- und β2a-Untereinheiten enthält.

6. Eine Zelle gemäß irgendeinem vorhergehenden Anspruch, wobei der einwärts gerichtete Gleichrichter-Kaliumkanal ein Kir2.3-Kanal ist.

7. Eine Zelle gemäß irgendeinem vorhergehenden Anspruch, ausgewählt aus: L-Zellen L-M(TK⁻) (ATCC CCL 1.3), L-Zellen L-M (ATCC CCL 1.2), HEK293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), CPAE (ATCC CCL 209), Saos-2 (ATCC HTB-85), ARPE-19 menschliches retinales Pigmentepithel (ATCC CRL-2302), GH3-Zellen, TREx-292-Zellen, T-REx-CHO-Zellen und primäre kardiale Myozyten.

8. Eine Zelle gemäß Anspruch 7, die eine HEK293-Zelle ist.

9. Ein Verfahren, um eine Verbindung auf Wirksamkeit als Agonist oder Antagonist eines Calciumkanals zu testen, umfassend die Schritte:
(a) Inkontaktbringen einer wie in irgendeinem vorhergehenden Anspruch definierten Zelle mit einer Lösung mit einer Kaliumkonzentration, wobei das Membranpotential der Zelle moduliert wird, ohne die Zelle vollständig zu depolarisieren,
(b) gleichzeitig mit oder im Anschluss an Schritt (a) Inkontaktbringen der Zelle mit (i) einer zu untersuchenden Substanz und (ii) einem Ion oder Molekül, das in der Lage ist, in die Zelle durch einen funktionellen Calciumkanal einzudringen,
(c) Depolarisieren der Zellmembran der Zelle,
(d) Erfassen des kanalvermittelten Ionenflusses in die Zelle und
(e) Vergleich des in Schritt (d) erfassten Ionenflusses mit einem in einem Kontrollversuch erzeugten Ionenflusses, wobei der Kontrollversuch das Unterwerfen einer separaten Zelle den Schritten (a), (b)(ii), (c) und (d), nicht jedoch Schritt (b)(i), umfasst,
wobei ein Unterschied zwischen dem in Schritt (d) erfassten Ionenfluss und dem des Kontrollversuchs zeigt, dass die zu untersuchende Substanz ein Agonist oder Antagonist eines Calciumkanals ist.

10. Ein Verfahren gemäß Anspruch 9 zur Identifizierung von zustandsabhängigen Antagonisten eines spannungsgesteuerten Calciumionenkanals, umfassend:
(a) Bereitstellen einer geteilten Gewebekulturplatte mit einzelnen Kammern, wobei wenigstens zwei der einzelnen Kammern eine Mehrzahl an lebenden eukaryotischen Zellen wie in irgendeinem der Ansprüche 1 bis 8 definiert enthalten, wobei das Zytoplasma der Zellen eine ionenempfindliche Fluoreszenzindikatorverbindung enthält,
(b) Einstellen des Membranpotentials der Zellen durch Verändern der extrazellulären Kaliumkonzentration in wenigstens einer der Kammern, welche die Zellen enthalten,
(c) Zugeben einer zu untersuchenden Substanz zu wenigstens einer der einzelnen Kammern, welche die Zellen enthalten,
(d) Depolarisieren der Zellen in den wenigstens zwei Kammern, welche Zellen enthalten, wobei wenigstens eine Kammer Schritt (c) unterworfen wird, Testgruppe, und wenigstens eine Kammer nicht Schritt (c) unterworfen wird, Kontrollgruppe,
(e) Erfassen des Ionenflusses in die Zellen von Schritt (d) und
(f) Vergleich des Ionenflusses in die Zellen der Testgruppe mit dem in die Zellen der Kontrollgruppe,
wobei, wenn der Wert für den Ionenfluss in den Testgruppenzellen niedriger ist als der in den Kontrollgruppenzellen, die Substanz ein Antagonist des spannungsgesteuerte Calciumionenkanals ist.

11. Das Verfahren nach Anspruch 9, ferner umfassend den Vergleich des Ionenflusses in der Testgruppe mit dem in einer zweiten Testgruppe, wobei die zweite Testgruppe Zellen enthält, die den Schritten (b) und (c) unterworfen wurden, deren Membranpotentiale jedoch auf einen Wert eingestellt wurden, der sich von dem der Testgruppenzellen unterscheidet,
wobei, wenn der Wert des Ionenflusses in den Testgruppenzellen sich von dem Wert des Ionenflusses in den Zellen der zweiten Testgruppe unterscheidet, die Substanz dann eine zustandsabhängige Wirkung auf den spannungsgesteuerten Calciumionenkanal besitzt.

12. Das Verfahren nach Anspruch 10 oder 11, wobei die geteilte Gewebekulturplatte eine Multiwell-Gewebekulturplatte ist, die wenigstens zwei Vertiefungen enthält.

13. Das Verfahren nach Anspruch 12, wobei die Multiwell-Gewebekulturplatte 12, 24, 96, 384, 1536 oder 3456 Vertiefungen enthält.

14. Das Verfahren nach irgendeinem der Ansprüche 9 bis 13, wobei wenigstens 10 Substanzen in einem Zeitraum von 24 Stunden getestet werden.

15. Das Verfahren nach irgendeinem der Ansprüche 10 bis 14, wobei die Fluoreszenzindikatorverbindung ausgewählt ist aus der Gruppe, bestehend aus Fluo-3, Fura-2, Fluo-4, Fluo-5, Calcium green-1, Oregon green, 488 BAPTA, SNARF-1 und Indo- 1.

16. Das Verfahren nach irgendeinem der Ansprüche 10 bis 15, wobei der Erfassungsschritt (e) ein Fluoreszenz- oder Lumineszenzmessgerät verwendet.

17. Das Verfahren nach irgendeinem der Ansprüche 10 bis 16, wobei der Erfassungsschritt (e) ein Fluorimetric-Imaging-Plate-Reader(FLIPR)- oder ein Voltage/Ion-Probe-Reader(VIPR)-Gerät verwendet.

## Revendications

1. Cellule eucaryote transformée pour exprimer un canal d'ions calcium potentiel-dépendant fonctionnel et un canal potassique avec rectificateur interne fonctionnel.

2. Cellule selon la revendication 1, dans laquelle les canaux sont exprimés sur la membrane plasmatique de la cellule.

3. Cellule selon la revendication 1 ou 2, dans laquelle le canal à ions calcium potentiel-dépendant est un canal calcique de type L.

4. Cellule selon la revendication 3, dans laquelle le canal calcique de type L comprend une sous-unité α1C.

5. Cellule selon la revendication 4, dans laquelle le canal calcique de type L est un complexe comprenant les sous-unités α1C, α2-δ et β2a.

6. Cellule selon l'une quelconque des revendications précédentes, dans laquelle le canal potassique à rectificateur interne est un canal Kir2.3.

7. Cellule selon l'une quelconque des revendications précédentes, qui est choisie parmi: les cellules L-M(TK⁻) (ATCC CCL 1.3), les cellules L L-M (ATCC CCL 1.2), HEK293 (ATCC CRL 1573), Raji (ATCC CCL 86), CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C127I (ATCC CRL 1616), BS-C-1 (ATCC CCL 26), MRC-5 (ATCC CCL 171), CPAE (ATCC CCL 209), Saos-2 (ATCC HTB-85), l'épithélium de pigment rétinien humain ARPE-19 (ATCC CRL-2302), les cellules GH3, les cellules TREx-292, les cellules T-REx-CHO et les myocytes cardiaques primaires.

8. Cellule selon la revendication 7, qui est une cellule HEK293.

9. Procédé pour tester un composé quant à l'activité en tant qu'agoniste ou antagoniste d'un canal calcique, comprenant les étapes consistant:
(a) à mettre en contact une cellule telle que définie dans l'une quelconque des revendications précédentes avec une solution ayant une concentration de potassium, le potentiel membranaire de la cellule étant modulé sans dépolariser totalement la cellule;
(b) simultanément avec ou après l'étape (a), à mettre en contact la cellule avec (i) une substance d'intérêt et (ii) un ion ou une molécule apte à pénétrer la cellule par un canal calcique fonctionnel;
(c) à dépolariser la membrane cellulaire de la cellule;
(d) à détecter le flux ionique pénétrant la cellule par l'intermédiaire des canaux; et
(e) à comparer le flux ionique ainsi détecté dans l'étape (d) avec un flux ionique produit dans une expérience témoin, l'expérience témoin comprenant la soumission d'une cellule séparée aux étapes (a), (b)(ii), (c) et (d), mais pas à l'étape (b)(i);
dans lequel une différence quant au flux ionique détecté dans l'étape (d) et l'expérience témoin indique que la substance d'intérêt est un agoniste ou antagoniste d'un canal calcique.

10. Procédé selon la revendication 9 pour identifier des antagonistes dépendant de l'état d'un canal d'ions calcium potentiel-dépendant, comprenant:
(a) la fourniture d'une plaque de culture tissulaire divisée comprenant des compartiments individuels, dans laquelle au moins deux des compartiments individuels contiennent une pluralité de cellules eucaryotes vivantes telles que définies dans l'une quelconque des revendications 1 à 8, le cytoplasme des cellules comprenant un composé indicateur fluorescent sensible aux ions;
(b) l'ajustement du potentiel de membrane des cellules en modifiant la concentration de potassium extracellulaire dans au moins l'un des compartiments contenant les cellules;
(c) l'addition d'une substance d'intérêt à au moins l'un des compartiments individuels contenant les cellules;
(d) la dépolarisation des cellules dans lesdits au moins deux compartiments contenant des cellules, dans laquelle au moins un compartiment est soumis à l'étape (c), le groupe d'essai, et au moins un compartiment n'est pas soumis à l'étape (c), le groupe témoin;
(e) la détection du flux ionique pénétrant les cellules de l'étape (d); et
(f) la comparaison du flux ionique pénétrant les cellules du groupe d'essai avec celui des cellules du groupe témoin;
dans lequel si la valeur du flux ionique des cellules du groupe d'essai est inférieure à celle des cellules du groupe témoin, la substance est un antagoniste du canal d'ions calcium potentiel-dépendant.

11. Procédé selon la revendication 9, comprenant en outre la comparaison du flux ionique dans le groupe d'essai avec celui d'un second groupe d'essai, le second groupe d'essai comprenant des cellules soumises aux étapes (b) et (c), mais dont les potentiels de membrane ont été ajustés à une valeur différente de celle des cellules du groupe d'essai;
dans lequel si la valeur du flux ionique des cellules du groupe d'essai est différente de la valeur du flux ionique des cellules du second groupe d'essai, alors la substance possède une puissance dépendante de l'état sur le canal d'ions calcium potentiel-dépendant.

12. Procédé selon la revendication 10 ou 11, dans lequel la plaque de culture tissulaire divisée est une plaque de culture tissulaire à puits multiples comprenant au moins deux puits.

13. Procédé selon la revendication 12, dans lequel la plaque de culture tissulaire à multiples puits comprend 12, 24, 96, 384, 1536 ou 3456 puits.

14. Procédé selon l'une quelconque des revendications 9 à 13, dans lequel au moins 10 substances sont testées en une période de 24 heures.

15. Procédé selon l'une quelconque des revendications 10 à 14, dans lequel le composé indicateur fluorescent est choisi dans le groupe constitué par fluo-3, fura-2, fluo-4, fluo-5, vert calcium-1, vert Orégon, 488 BAPTA, SNARF-1 et indo-1.

16. Procédé selon l'une quelconque des revendications 10 à 15, dans lequel l'étape de détection (e) emploie un dispositif indicateur de fluorescence ou de luminescence.

17. Procédé selon l'une quelconque des revendications 10 à 16, dans lequel l'étape de détection (e) emploie un dispositif à lecteur de plaque d'imagerie fluorimétrique (FLIPR) ou à lecteur de tension/sonde ionique (VIPR).
